# EUROPEAN PATENT APPLICATION

(11) **EP 4 563 026 A1**
(43) Date of publication of application: **04.06.2025**
(21) Application number: 22954006.7
(22) Date of filing: 04.08.2022
(51) Int. Cl.: A24F 40/90

(54) **AEROSOL GENERATION SYSTEM, CONTROL METHOD, AND PROGRAM**

(71) Applicant: Japan Tobacco Inc., Tokyo 105-6927 (JP)
(72) Inventor: SERITA, Kazutoshi, Tokyo 130-8603 (JP); TEZUKA, Hiroshi, Tokyo 130-8603 (JP)
(74) Representative: Hoffmann Eitle
(86) International application number: PCT/JP2022/029889
(87) International publication number: WO 2024/029017

(57) **Abstract**

[Problem] To provide a mechanism capable of further improving the quality of user's experiences using an inhalation device.

[Solution] An aerosol generation system comprising a first device, a second device and a controller, wherein: the first device has a heating unit that heats an aerosol source contained in a base material set in the first device on the basis of a heating profile that defines a time-series transition of parameters related to the temperature at which the aerosol source is heated, and a first power supply unit that supplies electric power to the heating unit; the second device has a second power supply unit that supplies electric power to the heating unit and/or the first power supply unit in the state where the first device and the second device are connected; and, when the heating unit executes heating in the state where the first device and the second device are connected, the controller chooses which of the first power supply unit or the second power supply unit will supply electric power to the heating unit on the basis of the electric power stored in the first power supply unit.

## Description

### Technical Field

The present disclosure relates to an aerosol generating system, a control method, and a program.

### Background Art

An inhaler device that generates material to be inhaled by a user, such as an electronic tobacco and a nebulizer, is widely used. For example, an inhaler device uses a substrate including an aerosol source for generating an aerosol, a flavor source for imparting a flavor component to the generated aerosol, and the like, to generate an aerosol with the imparted flavor component. The user is able to taste a flavor by inhaling the aerosol with the imparted flavor component, generated by the inhaler device. An action that a user takes to inhale an aerosol is also referred to as a puff or a puff action below.

With the aim of further improving the quality of user experience at the time of using such an inhaler device, various technologies are under development. For example, PTL 1 describes a technology for not performing heating operation when an inhaler device does not have an enough battery level at which heating of a single substrate can be completed.

### Citation List

### Patent Literature

Patent Literature 1: JP 5768192 B2

### Summary of Invention

### Technical Problem

It has only been a short period of time since the technology described in PTL 1 was developed, and there is still room for improvement in various viewpoints.

The present disclosure is contemplated in view of the above problem, and it is an object of the present disclosure to provide a mechanism of making it possible to further improve the quality of user experience using an inhaler device.

### Solution to Problem

To solve the above problem, an aspect of the present invention provides an aerosol generating system. The aerosol generating system includes a first device, a second device, and a control device. The first device includes a heater that heats an aerosol source included in a substrate set in the first device, in accordance with a heating profile defining time-series changes in parameter related to a temperature at which the aerosol source is heated, and a first power supply that supplies electric power to the heater. The second device includes a second power supply that supplies electric power to at least any one of the heater and the first power supply in a state where the first device and the second device are connected. When the heater performs heating in a state where the first device and the second device are connected, the control device selects a power supply source to the heater from between the first power supply and the second power supply in accordance with an amount of electric power stored in the first power supply.

When the amount of electric power stored in the first power supply is less than a threshold, the control device may select the second power supply as a power supply source to the heater in at least part of a period during which an operation of the heater is controlled in accordance with the heating profile.

In a period other than the at least part of the period during which the operation of the heater is controlled in accordance with the heating profile, the control device may select the first power supply as a power supply source to the heater when a predetermined condition is satisfied and select the second power supply as a power supply source to the heater when the predetermined condition is not satisfied.

The predetermined condition may include a condition that connection of the first device with the second device is released.

The predetermined condition may be a condition that the amount of electric power stored in the first power supply is greater than or equal to the threshold.

The period during which the operation of the heater is controlled in accordance with the heating profile may sequentially include an initial temperature rise period during which a temperature at which the aerosol source is heated increases from an initial temperature, an intermediate temperature fall period during which the temperature at which the aerosol source is heated decreases, and a re-temperature rise period during which the temperature at which the aerosol source is heated increases again. The at least part of the period may include the initial temperature rise period.

The control device may, before heating operation based on the heating profile, set the threshold to a value at which heating is allowed to be continued from a beginning to an end of the period during which the operation of the heater is controlled in accordance with the heating profile, and, during heating operation based on the heating profile, set the threshold to a value at which heating is allowed to be continued to an end of a remaining period of the period during which the operation of the heater is controlled in accordance with the heating profile.

The aerosol generating system may further include a notifier that notifies a user of information, and, when the amount of electric power stored in the first power supply is greater than or equal to the threshold, the notifier may notify information indicating that connection of the first device with the second device is allowed to be released.

The aerosol generating system may include the notifier provided in the first device and the notifier provided in the second device. When the first device and the second device are connected, the notifier provided in the second device may notify information indicating a status of the first device, and, when the first device and the second device are not connected, the notifier provided in the first device may notify information indicating a status of the first device.

The information indicating the status of the first device may relate to at least any one of a progress of heating operation with the heater, a state of charge of the first power supply, and an error having occurred in the first device.

When the second power supply is selected as a power supply source to the heater, the second power supply may supply electric power to both the heater and the first power supply.

In a state where the first device and the second device are connected, the control device may set in accordance with a user operation to the second device whether the second power supply supplies electric power to the first power supply, supplies electric power to the heater, or supplies electric power to both the first power supply and the heater.

In a state where the first device and the second device are connected, when the substrate is set in the first device, the second power supply may start supplying electric power to the heater.

To solve the above problem, another aspect of the present invention provides a control method. The control method is executed by a computer that controls at least any one of a first device and a second device. The first device includes a heater that heats an aerosol source included in a substrate set in the first device, in accordance with a heating profile defining time-series changes in parameter related to a temperature at which the aerosol source is heated, and a first power supply that supplies electric power to the heater. The second device includes a second power supply that supplies electric power to at least any one of the heater and the first power supply in a state where the first device and the second device are connected. The control method includes, when the heater performs heating in a state where the first device and the second device are connected, selecting a power supply source to the heater from between the first power supply and the second power supply in accordance with an amount of electric power stored in the first power supply.

To solve the above problem, another aspect of the present invention provides a program. The program is executed by a computer that controls at least any one of a first device and a second device. The first device includes a heater that heats an aerosol source included in a substrate set in the first device, in accordance with a heating profile defining time-series changes in parameter related to a temperature at which the aerosol source is heated, and a first power supply that supplies electric power to the heater. The second device includes a second power supply that supplies electric power to at least any one of the heater and the first power supply in a state where the first device and the second device are connected. The program causes the computer to, when the heater performs heating in a state where the first device and the second device are connected, select a power supply source to the heater from between the first power supply and the second power supply in accordance with an amount of electric power stored in the first power supply.

### Advantageous Effects of Invention

According to the present disclosure as described above, a mechanism of making it possible to further improve the quality of user experience using an inhaler device.

### Brief Description of Drawings

[Fig. 1] Fig. 1 is a view that illustrates an example of the external configuration of an aerosol generating system according to an embodiment of the present disclosure.
[Fig. 2] Fig. 2 is a view that illustrates an example of a state where a stick substrate is inserted in an inhaler device according to the present embodiment.
[Fig. 3] Fig. 3 is a view that illustrates an example of a state where connection of the inhaler device with a charging device is released according to the present embodiment.
[Fig. 4] Fig. 4 is a schematic diagram that schematically illustrates a configuration example of the inhaler device.
[Fig. 5] Fig. 5 is a schematic diagram that schematically illustrates a configuration example of the charging device.
[Fig. 6] Fig. 6 is a view for illustrating an example of airflow that is generated in the inhaler device as a result of puffing according to the present embodiment.
[Fig. 7] Fig. 7 is a view that illustrates an example of the configuration of a bottom of a container according to the present embodiment.
[Fig. 8] Fig. 8 is a view that schematically illustrates an example of the configuration of an inner wall of the container according to the present embodiment.
[Fig. 9] Fig. 9 is a view that schematically illustrates an example of the configuration of the inner wall of the container according to the present embodiment.
[Fig. 10] Fig. 10 is a view for illustrating another example of airflow that is generated in the inhaler device as a result of puffing according to the present embodiment.
[Fig. 11] Fig. 11 is a view for illustrating another example of airflow that is generated in the inhaler device as a result of puffing according to the present embodiment.
[Fig. 12] Fig. 12 is a view for illustrating an example of arrangement of a proximity sensor in the inhaler device according to the present embodiment.
[Fig. 13] Fig. 13 is a graph that schematically illustrates an example of a heating profile according to the present embodiment.
[Fig. 14] Fig. 14 is a flowchart that illustrates an example of the flow of a process that is executed by the aerosol generating system according to the present embodiment.
[Fig. 15] Fig. 15 is a graph that schematically illustrates an example of a heating profile according to the present embodiment.
[Fig. 16] Fig. 16 is a graph that schematically illustrates an example of switching of the heating profile according to the present embodiment.
[Fig. 17] Fig. 17 is a view for illustrating an example of information that is notified by an LED of the charging device according to the present embodiment.
[Fig. 18] Fig. 18 is a view for illustrating an example of information that is notified by the LED of the charging device according to the present embodiment.
[Fig. 19] Fig. 19 is a view for illustrating an example of information that is notified by the LED of the charging device according to the present embodiment.
[Fig. 20] Fig. 20 is a view for illustrating an example of information that is notified by the LED of the charging device according to the present embodiment.
[Fig. 21] Fig. 21 is a view for illustrating an example of information that is notified by the LED of the charging device according to the present embodiment.
[Fig. 22] Fig. 22 is a view for illustrating a first example of an attachment and detachment mechanism of an inhaler device according to a first modification.
[Fig. 23] Fig. 23 is a view that schematically illustrates an example of a cross section of the inhaler device in a state where connection of a cap with a body is released in the first example of the attachment and detachment mechanism.
[Fig. 24] Fig. 24 is a view that schematically illustrates an example of a cross section of the inhaler device in a state where the cap and the body are connected in the first example of the attachment and detachment mechanism.
[Fig. 25] Fig. 25 is a view that schematically illustrates another example of a cross section of the inhaler device in a state where the cap and the body are connected in the first example of the attachment and detachment mechanism.
[Fig. 26] Fig. 26 is a view for illustrating a second example of the attachment and detachment mechanism of the inhaler device according to the first modification.
[Fig. 27] Fig. 27 is a view that schematically illustrates an example of a cross section of the inhaler device in a state where connection of a cap with a body is released in the second example of the attachment and detachment mechanism.
[Fig. 28] Fig. 28 is a view that schematically illustrates an example of a cross section of the inhaler device in a state where the cap and the body are connected in the second example of the attachment and detachment mechanism.
[Fig. 29] Fig. 29 is a view that schematically illustrates another example of a cross section of the inhaler device in a state where the cap and the body are connected in the second example of the attachment and detachment mechanism.
[Fig. 30] Fig. 30 is a view for illustrating the outline of an aerosol generating system according to a second modification.
[Fig. 31] Fig. 31 is a flowchart that illustrates an example of the flow of a process that is executed by an inhaler device according to the modification.
[Fig. 32] Fig. 32 is a schematic diagram that schematically illustrates an example of the configuration of an inhaler device according to a third modification.
[Fig. 33] Fig. 33 is a graph that schematically illustrates examples of heating profiles according to the modification.
[Fig. 34] Fig. 34 is a graph that schematically illustrates examples of heating profiles according to the modification.
[Fig. 35] Fig. 35 is a schematic diagram that schematically illustrates a configuration example of an inhaler device according to a fourth modification.
[Fig. 36] Fig. 36 is a view for illustrating the outline of an aerosol generating system according to a fifth modification.

### Description of Embodiments

Hereinafter, a preferred embodiment of the present disclosure will be described in detail with reference to the attached drawings. In the specification and the drawings, like reference signs denote structural elements having substantially the same functional configurations, and the description will not be repeated.

In the specification and the drawings, elements each having substantially the same functional configuration can be distinguished from one another by suffixing "-" and different alphabets to the same reference signs. For example, a plurality of elements each having substantially the same functional configuration is distinguished from one another like a heater 121-1 and a heater 121-2 where necessary. However, when a plurality of elements each having substantially the same functional configuration does not need to be distinguished from one another, only the same reference sign is assigned. When, for example, the heater 121-1 and the heater 121-2 do not need to be distinguished from each other, the heaters 121-1, 121-2 are simply referred to as heaters 121.

### 1. Embodiment

### 1.1. System configuration

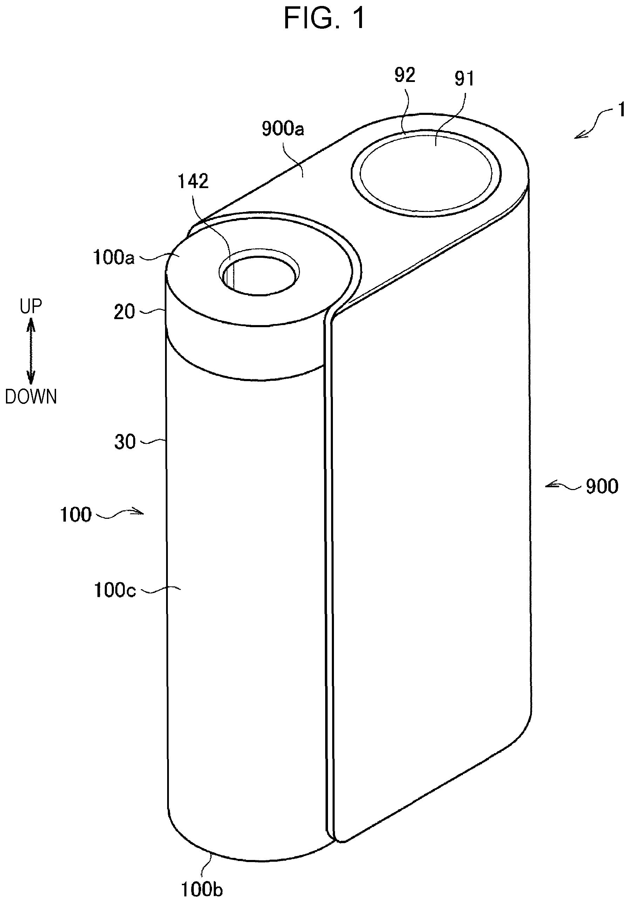

Initially, the outline of an aerosol generating system according to an embodiment of the present disclosure will be described with reference to Figs. 1 to 3.

Fig. 1 is a view that illustrates an example of the external configuration of the aerosol generating system according to the embodiment of the present disclosure. As illustrated in Fig. 1, the aerosol generating system 1 according to the present embodiment includes an inhaler device 100 and a charging device 900. The inhaler device 100 is a device that generates material to be inhaled by a user. Hereinafter, the description will be made on the assumption that the material to be generated by the inhaler device is an aerosol. Alternatively, the material to be generated by the inhaler device may be gas. The charging device 900 is a device that supplies electric power to another device. In an example, the charging device 900 supplies electric power to the inhaler device 100 and charges the inhaler device 100.

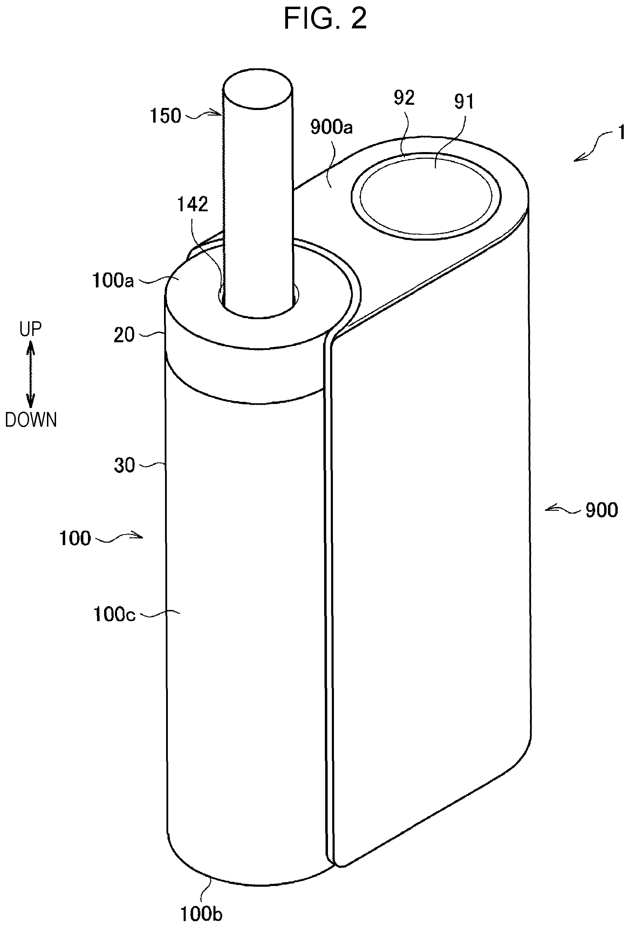

Fig. 2 is a view that illustrates an example of a state where a stick substrate 150 is inserted in the inhaler device 100 according to the present embodiment. As illustrated in Fig. 2, the stick substrate 150 can be inserted into the inhaler device 100 through an opening 142 provided at a top surface 100a of the inhaler device 100. The stick substrate 150 is an example of a substrate including an aerosol source. The inhaler device 100 generates an aerosol by heating the aerosol source included in the stick substrate 150. A combination of the inhaler device 100, the charging device 900, and the stick substrate 150 may be regarded as the aerosol generating system 1.

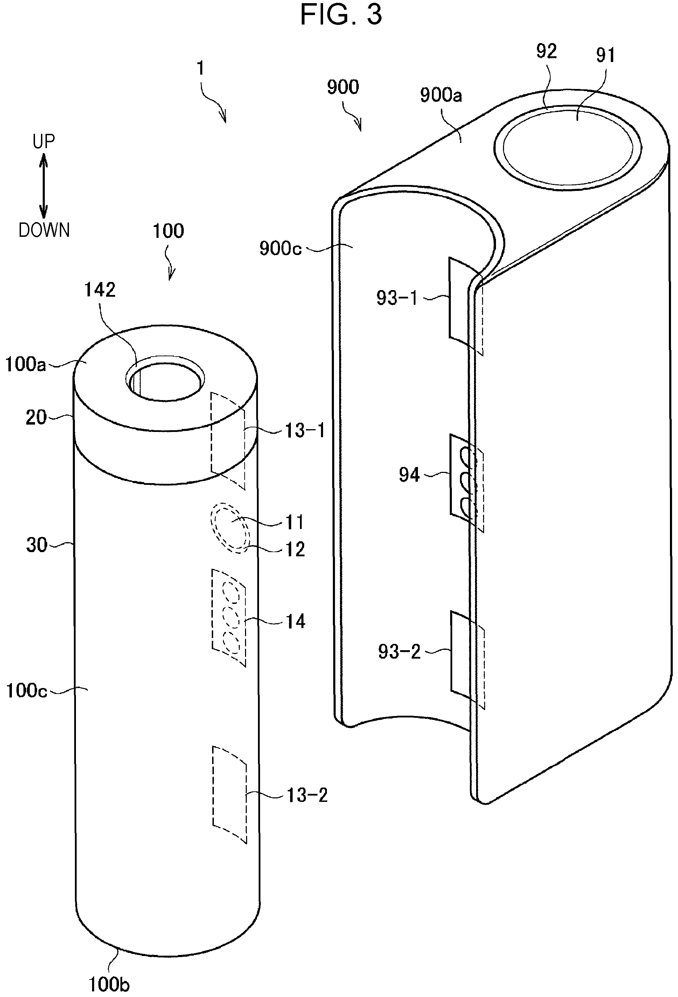

Fig. 3 is a view that illustrates an example of a state where connection of the inhaler device 100 with the charging device 900 is released according to the present embodiment. As illustrated in Fig. 3, the inhaler device 100 and the charging device 900 may be configured to be detachable. The stick substrate 150 may be heated and an aerosol may be generated in a state where the inhaler device 100 and the charging device 900 are connected. The stick substrate 150 may be heated and an aerosol may be generated in a state where connection of the inhaler device 100 with the charging device 900 is released.

As illustrated in Figs. 1 to 3, a longitudinal direction of the inhaler device 100 is also referred to as up and down direction. A downward direction corresponds to an insertion direction of the stick substrate 150. An upward direction corresponds to a removal direction of the stick substrate 150. In a casing of the aerosol generating system 1, a surface facing in the upward direction is also referred to as top surface, a surface facing in the downward direction is also referred to as bottom surface, and a surface facing in a direction orthogonal to the up and down direction is also referred to as side surface.

As illustrated in Figs. 1 to 3, the inhaler device 100 includes a cap 20 and a body 30. The body 30 is an example of a first component having structural elements of the inhaler device 100, that is, a heater 121, a controller 116, and the like (described later). The cap 20 is an example of a second component that is detachably connected to the body 30. The cap 20 may be an accessory configured such that part of an outer shell of the inhaler device 100 is removable. For example, the cap 20 is attached to the body 30 so as to wrap around an upper end of the body 30. For example, a user may have a plurality of caps 20 having different colors. A user is able to adjust the appearance of the inhaler device 100 by changing the cap 20 according to his or her feeling. Of course, the cap 20 and the body 30 may be formed integrally.

As illustrated in Fig. 3, the inhaler device 100 is formed in a circular columnar shape such that a top surface 100a and a bottom surface 100b are both ends. Then, a button 11 is provided at a side surface 100c of the inhaler device 100. The button 11 is an example of an operating portion capable of receiving a user operation to the aerosol generating system 1. In an example, when the button 11 is depressed, the inhaler device 100 may start heating the stick substrate 150.

As illustrated in Fig. 3, a light-emitting diode (LED) 12 is provided at the side surface 100c of the inhaler device 100. The LED 12 is disposed in association with the button 11. Specifically, the LED 12 is disposed so as to surround the button 11. The LED 12 is an example of the notifier that outputs information to be notified to a user from the aerosol generating system 1. The LED 12 may notify information on a process executed in response to depression of the button 11 as a trigger. In an example, the LED 12 may output information indicating the progress of heating operation of the stick substrate 150 with the inhaler device 100. With the above configuration, it is possible to further clarify the relationship between user operation and notification.

As illustrated in Fig. 3, a button 91 is provided at a top surface 900a of the charging device 900. The button 91 is an example of the operating portion capable of receiving a user operation to the aerosol generating system 1. In an example, when the button 91 is depressed, the charging device 900 may start or stop charging the inhaler device 100.

As illustrated in Fig. 3, an LED 92 is provided at the top surface 900a of the charging device 900. The LED 92 is disposed in association with the button 91. Specifically, the LED 92 is disposed so as to surround the button 91. The LED 92 is an example of the notifier that outputs information to be notified to a user from the aerosol generating system 1. The LED 92 may notify information on a process executed in response to depression of the button 91 as a trigger. In an example, the LED 92 may output information indicating the progress of charging of the inhaler device 100. With the above configuration, it is possible to further clarify the relationship between user operation and notification.

As illustrated in Fig. 3, a recessed surface 900c is provided at a side surface of the charging device 900. The recessed surface 900c has a shape formed along the side surface 100c of the inhaler device 100. Then, in a state where the recessed surface 900c is in contact with the side surface 100c of the inhaler device 100, the inhaler device 100 and the charging device 900 are connected. A magnetic portion 13-1 and a magnetic portion 13-2 are provided at the side surface 100c of the inhaler device 100. On the other hand, a magnetic portion 93-1 and a magnetic portion 93-2 are provided at the recessed surface 900c of the charging device 900. The magnetic portion 13-1, the magnetic portion 13-2, the magnetic portion 93-1, and the magnetic portion 93-2 are bodies that generate magnetic fields and are, for example, magnets. When the inhaler device 100 and the charging device 900 are brought close to each other, the magnetic portion 13-1 and the magnetic portion 93-1 attract each other, and the magnetic portion 13-2 and the magnetic portion 93-2 attract each other, with the result that the inhaler device 100 and the charging device 900 are connected. In an example, one of the magnetic portion 13-1 and the magnetic portion 93-1 may be an S-pole magnet, and the other may be an N-pole magnet. Similarly, one of the magnetic portion 13-2 and the magnetic portion 93-2 may be an S-pole magnet, and the other may be an N-pole magnet. With the above configuration, the inhaler device 100 and the charging device 900 can be connected so as to be easily attached and removed.

As illustrated in Fig. 3, an electrical contact 14 is provided at the side surface 100c of the inhaler device 100 between the magnetic portion 13-1 and the magnetic portion 13-2. The electrical contact 14 is a contact to an electrical circuit in the inhaler device 100. On the other hand, an electrical contact 94 is provided at the recessed surface 900c of the charging device 900 between the magnetic portion 93-1 and the magnetic portion 93-2. The electrical contact 94 is a contact to an electrical circuit in the charging device 900. In a state where the inhaler device 100 and the charging device 900 are connected, the electrical contact 14 and the electrical contact 94 contact with each other. Then, the charging device 900 supplies electricity to the inhaler device 100 via the electrical contact 14 and the electrical contact 94. Thus, the inhaler device 100 can be charged. Connecting the inhaler device 100 to the charging device 900 includes not only physical connection but also electrical connection.

Charging from the charging device 900 to the inhaler device 100 may be started or stopped in response to connection of the inhaler device 100 to the charging device 900 or release of connection as a trigger. Physical connection of the inhaler device 100 to the charging device 900 or release of connection can be detected by a magnetic sensor capable of detecting a magnetic field that is generated from the magnetic portion 93 and/or the magnetic portion 13. Examples of the magnetic sensor include a Hall sensor. Electrical connection of the inhaler device 100 to the charging device 900 or release of connection can be detected in accordance with whether energization is possible via the electrical contact 14 and the electrical contact 94.

Typically, a user removes the inhaler device 100 from the charging device 900 and uses the inhaler device 100. Unless otherwise mentioned, using the pulling device 100 by a user means inhaling an aerosol by heating the stick substrate 150 with the inhaler device 100. On the other hand, a user may use the inhaler device 100 while the inhaler device 100 remains connected to the charging device 900. In this case, it is possible to use the inhaler device 100 without concern for a battery level of the inhaler device 100.

As illustrated in Fig. 3, the button 11 is disposed in the same line as the magnetic portion 13-1, the magnetic portion 13-2, and the electrical contact 14. With the above configuration, the button 11 is hidden by the recessed surface 900c of the charging device 900 in a state where the inhaler device 100 and the charging device 900 are connected. Thus, it is possible to prevent an erroneous operation to the button 11.

Similarly, as illustrated in Fig. 3, the LED 12 is disposed in the same line as the magnetic portion 13-1, the magnetic portion 13-2, and the electrical contact 14. With the above configuration, the LED 12 is hidden by the recessed surface 900c of the charging device 900 in a state where the inhaler device 100 and the charging device 900 are connected. Thus, it is possible to aggregate sources to notify information to the LED 92.

The inhaler device 100 and the charging device 900 may transmit and receive information via the electrical contact 14 and the electrical contact 94. Examples of the information that is transmitted from the inhaler device 100 to the charging device 900 include inhalation information and biometric information, acquired by the inhaler device 100. On the other hand, examples of the information that is transmitted from the charging device 900 to the inhaler device 100 include control information for instructing the inhaler device 100 to execute a predetermined process. The inhalation information is information acquired at the time when a user heats an aerosol with the inhaler device 100. Examples of the inhalation information include the number of puffs, the number of stick substrates 150 heated, and the frequency of heating of the stick substrate 150. Examples of the biometric information include the blood pressure, pulse, and body temperature of a user. The charging device 900 may output information acquired from the inhaler device 100 via the LED 92 or an output device incorporated in the charging device 900, such as a display (not illustrated), or may transmit the information to another device, such as a smartphone. Of course, the inhaler device 100 may output these pieces of information via the LED 12 or an output device incorporated in the inhaler device 100, such as a display (not illustrated).

The inhaler device 100 typically operates in accordance with a user operation to the inhaler device 100. For example, the inhaler device 100 starts or stops heating the stick substrate 150 in accordance with depression of the button 11. Alternatively, the inhaler device 100 may operate in accordance with a user operation to the charging device 900. For example, in a state where the inhaler device 100 is connected to the charging device 900, the inhaler device 100 may start or stop heating the stick substrate 150 in accordance with depression of the button 91. In consideration of the fact that the button 11 is hidden and it is difficult to depress the button 11 in a state where the inhaler device 100 and the charging device 900 are connected, it is possible to improve usability with the above configuration.

The outline of the aerosol generating system 1 has been described. The configuration of the aerosol generating system 1 is not limited to the example described above. Various modifications illustrated below may be applied.

In an example, the inhaler device 100 and the charging device 900 may be configured so as to be not detachable. In other words, the inhaler device 100 and the charging device 900 may be formed integrally.

In another example, the number of the magnetic portions 13 provided in the inhaler device 100 is not limited to two and may be one or may be three or more. Similarly, the number of the electrical contacts 14 provided in the inhaler device 100 is not limited to one and may be two or more. The positions of the magnetic portions 13 provided in the inhaler device 100 are not limited to the side surface 100c of the inhaler device 100 and may be the bottom surface 100b of the inhaler device 100. Similarly, the position of the electrical contact 14 provided in the inhaler device 100 is not limited to the inhaler device 100 and may be the bottom surface 100b of the inhaler device 100. The magnetic portions 93 and the electrical contact 94 just need to be provided in the charging device 900 in numbers and positions corresponding to the numbers and positions of the magnetic portions 13 and electrical contact 14 provided in the inhaler device 100.

In another example, the electrical contact 14 may have a function as the magnetic portion 13. Similarly, the electrical contact 94 may have a function as the magnetic portion 93.

In another example, the charging device 900 may store the whole of the inhaler device 100. For example, a circular columnar space for storing the inhaler device 100 may be provided inside the charging device 900, and the inhaler device 100 may be accommodated in the space. A space capable of accommodating the inhaler device 100 may be opened and closed with a lid provided at a selected position of an outer shell of the charging device 900.

In another example, the charging device 900 may wirelessly charge the inhaler device 100. In this case, the electrical contact 14 and the electrical contact 94 may be omitted.

### 1.2. Logical configuration

### (1) Configuration example of inhaler device 100

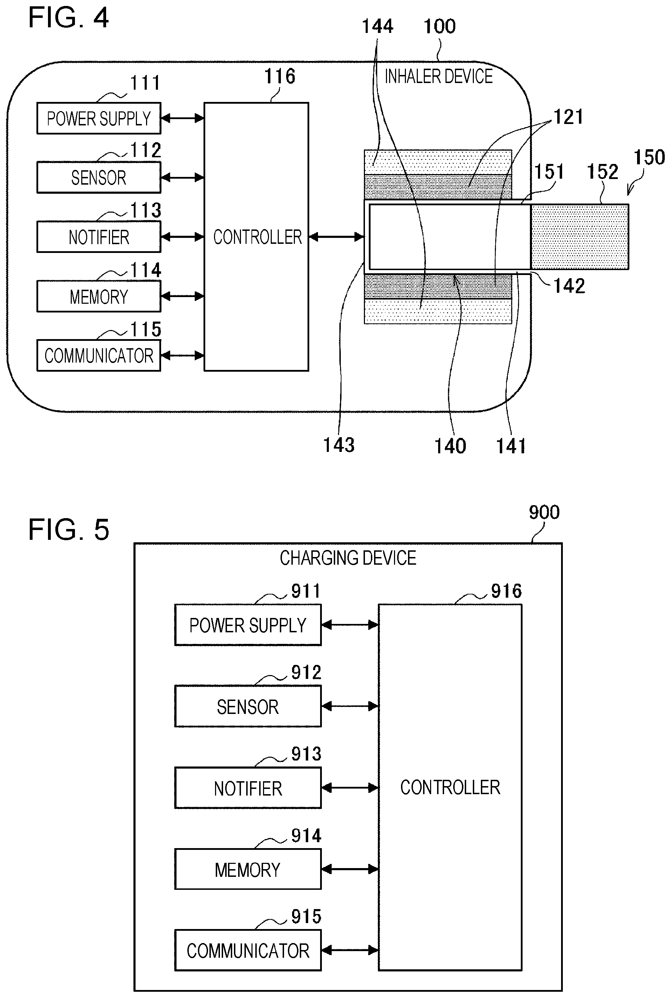

Fig. 4 is a schematic diagram that schematically illustrates a configuration example of the inhaler device 100. As illustrated in Fig. 4, the inhaler device 100 according to the present configuration example includes a power supply 111, a sensor 112, a notifier 113, a memory 114, a communicator 115, a controller 116, a heater 121, a container 140, and a heat insulator 144.

The power supply 111 stores electric power. The power supply 111 supplies electric power to the structural elements of the inhaler device 100 under the control of the controller 116. The power supply 111 can be a rechargeable battery, such as a lithium ion secondary battery.

The sensor 112 acquires various items of information regarding the inhaler device 100. In an example, the sensor 112 may be a pressure sensor such as a capacitor microphone, a flow sensor, or a temperature sensor, and acquire a value generated in accordance with user's inhalation. In another example, the sensor 112 is an input device that receives information input by a user, such as a button and a switch.

The notifier 113 notifies a user of information. The notifier 113 may be a light-emitting device that emits light, a display device that displays an image, a sound output device that outputs sound, or a vibration device that vibrates.

The memory 114 stores various items of information for the operation of the inhaler device 100. The memory 114 is, for example, a non-volatile storage medium, such as a flash memory.

The communicator 115 is a communication interface capable of communication in conformity with any wired or wireless communication standard. For example, a standard using Wi-Fi (registered trademark), Bluetooth (registered trademark), Bluetooth Low Energy (BLE) (registered trademark), near field communication (NFC), or a low-power wide area (LPWA) can be adopted as such a communication standard.

The controller 116 functions as an arithmetic processing unit and a control device and controls the overall operations in the inhaler device 100 in accordance with various programs. The controller 116 is implemented by, for example, an electronic circuit, such as a central processing unit (CPU) and a microprocessor.

The container 140 has an internal space 141. The container 140 holds the stick substrate 150 while accommodating part of the stick substrate 150 in the internal space 141. The container 140 has the opening 142 that allows the internal space 141 to communicate with outside. The container 140 accommodates the stick substrate 150 that is inserted into the internal space 141 through the opening 142. For example, the container 140 is a tubular body having the opening 142 and a bottom 143 at its ends, and defines the pillar-shaped internal space 141. An airflow path for supplying air to the internal space 141 is connected to the container 140. An air inlet hole that is an inlet for air into the airflow path is disposed at, for example, the side surface of the inhaler device 100. An air outlet hole that is an outlet for air from the airflow path to the internal space 141 is disposed at, for example, the bottom 143.

The stick substrate 150 includes a substrate 151 and an inhalation port 152. The substrate 151 includes an aerosol source. The aerosol source includes a flavor component that is either derived from tobacco or not derived from tobacco. For the inhaler device 100 that is a medical inhaler such as a nebulizer, the aerosol source may include a medicine. The aerosol source may be, for example, a liquid, such as polyhydric alcohol and water, containing a flavor component derived from tobacco or not derived from tobacco. Examples of the polyhydric alcohol include glycerin and propylene glycol. The aerosol source may also be a solid containing a flavor component derived from tobacco or not derived from tobacco. The stick substrate 150 held by the container 140 includes the substrate 151 at least partially accommodated in the internal space 141 and the inhalation port 152 at least partially protruding from the opening 142. When a user inhales with the inhalation port 152 protruding from the opening 142 in his/her mouth, air flows into the internal space 141 through the airflow path (not illustrated), and the air and an aerosol generated from the substrate 151 reach the inside of the mouth of the user.

The heater 121 heats the aerosol source to atomize the aerosol source and generate the aerosol. In the example illustrated in Fig. 4, the heater 121 has a film-like shape and surrounds the outer circumference of the container 140. Subsequently, heat produced from the heater 121 heats the substrate 151 of the stick substrate 150 from the outer circumference, and an aerosol is generated. The heater 121 produces heat when receiving electric power from the power supply 111. In an example, the electric power may be supplied in response to the sensor 112 detecting a start of the user's inhalation and/or an input of predetermined information. Subsequently, the supply of the electric power may be stopped in response to the sensor 112 detecting an end of the user's inhalation and/or an input of predetermined information.

The heat insulator 144 prevents heat from transferring from the heater 121 to the other structural elements. For example, the heat insulator 144 may be a vacuum heat insulator or an aerogel heat insulator.

The configuration example of the inhaler device 100 has been described above. The inhaler device 100 is not limited to the above configuration, and may be configured in various ways as exemplified below.

In an example, the heater 121 may have a blade-like shape, and may be disposed such that the heater 121 protrudes from the bottom 143 of the container 140 toward the internal space 141. In this case, the heater 121 having the blade-like shape is inserted into the substrate 151 of the stick substrate 150 and heats the substrate 151 of the stick substrate 150 from its inside. In another example, the heater 121 may be disposed such that the heater 121 covers the bottom 143 of the container 140. In still another example, the heater 121 may be implemented as a combination of two or more selected from among a first heater that covers the outer circumference of the container 140, a second heater having the blade-like shape, and a third heater that covers the bottom 143 of the container 140.

In another example, the container 140 may include an opening/closing mechanism that at least partially opens and closes an outer shell defining the internal space 141. Examples of the opening/closing mechanism include a hinge. In addition, the container 140 may accommodate the stick substrate 150 inserted into the internal space 141 while sandwiching the stick substrate 150, by opening and closing the outer shell. In this case, the heater 121 may be at the sandwiching position of the container 140 and may produce heat while pressing the stick substrate 150.

In addition, means for atomizing the aerosol source is not limited to heating with the heater 121. For example, the means for atomizing the aerosol source may be induction heating. In this case, the inhaler device 100 may include at least an electromagnetic induction source, such as a coil, that generates a magnetic field, instead of the heater 121. A susceptor that produces heat by using induction heating may be provided in the inhaler device 100 or may be included in the stick substrate 150.

### (2) Configuration example of charging device 900

Fig. 5 is a schematic diagram that schematically illustrates a configuration example of the charging device 900. As illustrated in Fig. 5, the charging device 900 according to the present configuration example includes a power supply 911, a sensor 912, a notifier 913, a memory 914, a communicator 915, and a controller 916.

The power supply 911 stores electric power. The power supply 911 supplies electric power to the structural elements of the charging device 900 under the control of the controller 916. The power supply 911 supplies electric power to the inhaler device 100 connected to the charging device 900. The power supply 911 can be a rechargeable battery, such as a lithium ion secondary battery.

The sensor 912 acquires various items of information regarding the charging device 900. In an example, the sensor 912 detects connection of the inhaler device 100 to the charging device 900 and release of connection. In an example, the sensor 912 is an input device that receives information input by a user, such as a button and a switch.

The notifier 913 notifies a user of information. The notifier 913 may be, for example, a light-emitting device that emits light, a display device that displays an image, a sound output device that outputs sound, a vibration device that vibrates, or the like.

The memory 914 stores various items of information for the operation of the charging device 900. The memory 914 is, for example, a non-volatile storage medium, such as a flash memory.

The communicator 915 is a communication interface capable of communication in conformity with any wired or wireless communication standard. For example, a standard using Wi-Fi (registered trademark), Bluetooth (registered trademark), Bluetooth Low Energy (BLE) (registered trademark), near field communication (NFC), or a low-power wide area (LPWA) can be adopted as such a communication standard.

The controller 916 functions as an arithmetic processing unit and a control device and controls the overall operations in the charging device 900 in accordance with various programs. The controller 916 is implemented by, for example, an electronic circuit, such as a central processing unit (CPU) and a microprocessor.

### (3) Supplement

The inhaler device 100 is an example of the first device. The heater 121 is an example of the heater that heats an aerosol source included in the stick substrate 150 set in the inhaler device 100. A substrate including an aerosol source is not limited to the stick substrate 150 formed in a stick shape and can have various shapes, such as a card shape and a capsule shape. A substrate including an aerosol source may be set in the inhaler device 100 in a system different from insertion, such as accommodating the whole of a substrate in the inhaler device 100. The power supply 111 is an example of the first power supply that supplies electric power to the heater 121.

The charging device 900 is an example of the second device. The power supply 911 of the charging device 900 is an example of the second power supply. The power supply 911 of the charging device 900 supplies electric power to the inhaler device 100 in a state where the inhaler device 100 and the charging device 900 are connected. The inhaler device 100 may heat the stick substrate 150 by using electric power supplied from the charging device 900, may charge the power supply 111, or may concurrently perform these operations.

A correspondence relation between the structural elements described with reference to Figs. 1 to 3 and the structural elements described with reference to Figs. 4 and 5 will be described below. The button 11 is an example of the input device included in the sensor 112. The button 91 is an example of the input device included in the sensor 912. The LED 12 is an example of the notifier 113. The LED 92 is an example of the notifier 913. The electrical contact 14 and the electrical contact 94 are an example of an electrical circuit that connects the power supply 111 or the heater 121 to the power supply 911. The electrical contact 14 and the electrical contact 94 are an example of a communication path between the communicator 115 and the communicator 915. The sensor 912 may have a magnetic sensor that detects a magnetic field that is generated from the magnetic portion 13-1 and the magnetic portion 13-2.

The controller 116 of the inhaler device 100 and the controller 916 of the charging device 900 are examples of the control device that controls the operations of the aerosol generating system 1. Various processes that are executed by the aerosol generating system 1 may be executed in accordance with the control of the controller 116 of the inhaler device 100 or may be executed in accordance with the control of the controller 916 of the charging device 900. In other words, the inhaler device 100 may operate in accordance with the control of the controller 916 of the charging device 900. The charging device 900 may operate in accordance with the control of the controller 116 of the inhaler device 100. Information for controlling various processes can be transmitted between the inhaler device 100 and the charging device 900. In an example, the controller 116 of the inhaler device 100 may control a process that is executed by the inhaler device 100 or the charging device 900, in accordance with information received from the charging device 900 via the communicator 115. In another example, the controller 916 of the charging device 900 may control a process that is executed by the inhaler device 100 or the charging device 900, in accordance with information received from the inhaler device 100 via the communicator 915.

Hereinafter, for the sake of convenience, there are cases where the description will be made on the assumption that the controller 116 or the controller 916 is a subject of control; however, a control subject may be any one of the controller 116 and the controller 916. In other words, a process described to be controlled by the controller 116 may be controlled by the controller 916. Similarly, a process described to be controlled by the controller 916 may be controlled by the controller 116.

### 1.3. Airflow

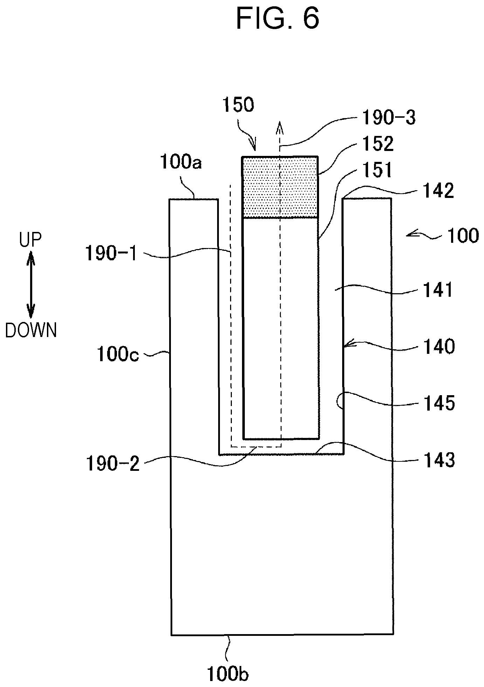

Hereinafter, airflow that is generated in the inhaler device 100 as a result of puffing according to the present embodiment will be described with reference to Figs. 6 to 8.

Fig. 6 is a view for illustrating an example of airflow that is generated in the inhaler device 100 as a result of puffing according to the present embodiment. Fig. 6 schematically illustrates an example of a cross section of the inhaler device 100 and the stick substrate 150 inserted in the inhaler device 100, taken in the up and down direction so as to pass through the center of the container 140. As illustrated in Fig. 6, in a state where the stick substrate 150 is accommodated in the container 140, an air gap is present between the stick substrate 150 and the bottom 143 and inner wall 145 of the container 140. When a user inhales with the stick substrate 150 in his or her mouth, air flowing in through the opening 142 passes through the air gap and flows into the stick substrate 150 from the distal end of the substrate 151 and then flows out from the rear end of the inhalation port 152 into the mouth of the user. In other words, air inhaled by a user flows in order of airflow 190-1, airflow 190-2, and airflow 190-3 and is introduced into the oral cavity of the user in a state of being mixed with an aerosol generated from the stick substrate 150. With the airflow 190-1, the airflow 190-2, and the airflow 190-3, air flows into the internal space 141 through the opening 142, and air flows out through the opening 142. In this way, a mode of suction and discharge in which a suction passage and a discharge passage are the same is also referred to as counter flow.

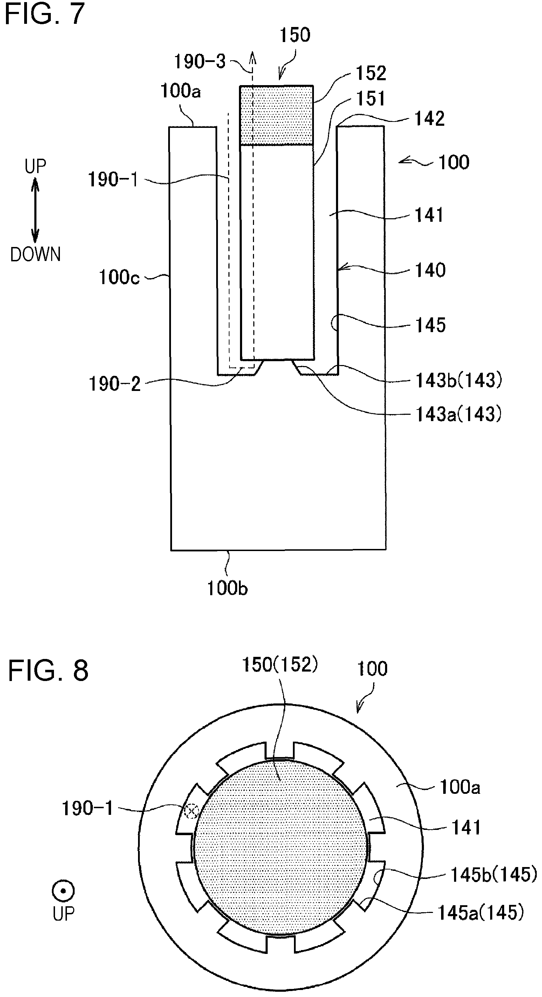

Fig. 7 is a view that illustrates an example of the configuration of the bottom 143 of the container 140 according to the present embodiment. Fig. 7 schematically illustrates an example of a cross section of the inhaler device 100 and the stick substrate 150 inserted in the inhaler device 100, taken in the up and down direction of the container 140. As illustrated in Fig. 7, a projection 143a that projects toward the internal space 141 may be provided at the bottom 143 of the container 140. A part of the bottom 143, other than the projection 143a, is also referred to as recess 143b. The projection 143a has, for example, a truncated cone shape with a planar top surface. A top surface of the projection 143a is formed so as to be at least smaller than an end surface of the stick substrate 150. Thus, as illustrated in Fig. 7, the projection 143a can support the stick substrate 150 in a state where at least part of the end surface of the stick substrate 150 is spaced apart from the recess 143b. As a result, an air gap through which the airflow 190-2 passes can be formed between the end surface of the stick substrate 150 and the recess 143b. It is also possible to connect the airflow 190-2 with the airflow 190-3 without a hindrance.

Fig. 8 is a view that illustrates an example of the configuration of the inner wall 145 of the container 140 according to the present embodiment. Fig. 8 schematically illustrates an example of a state where the inhaler device 100 and the stick substrate 150 inserted in the inhaler device 100 are viewed from above. As illustrated in Fig. 8, eight projections 145a that project toward the internal space 141 may be provided at the inner wall 145 of the container 140 at equal intervals in a circumferential direction. Parts of the inner wall 145, other than the projections 145a, are also referred to as recesses 145b. The projections 145a are provided so as to span over the entire region in the up and down direction from the opening 142 to the bottom 143. The recesses 145b are also provided so as to span over the entire region in the up and down direction from the opening 142 to the bottom 143. In other words, the cross-sectional shape of the inner wall 145 in a plane orthogonal to the up and down direction is the same as the shape of the inner wall 145 when viewed from above, illustrated in Fig. 8. Thus, as illustrated in Fig. 8, the stick substrate 150 can be supported in a state where at least part of the side surface of the stick substrate 150 is spaced apart from the recesses 145b. As a result, an air gap through which the airflow 190-1 passes can be formed between the side surface of the stick substrate 150 and the recesses 145b. The recesses 145b provided at the inner wall 145 are connected with the recess 143b provided at the bottom 143. As a result, it is also possible to connect the airflow 190-1 with the airflow 190-2 without a hindrance.

The projections 145a may hold the stick substrate 150 so as to press the stick substrate 150. In an example, in the plane orthogonal to the up and down direction, the width between the opposite pairs of projections 145a may be formed so as to be less than or equal to the width of the stick substrate 150. With the above configuration, the container 140 can sandwich the inserted stick substrate 150 in multiple directions while pressing the stick substrate 150 with the opposite pairs of projections 145a.

Airflow that is generated in the inhaler device 100 as a result of puffing has been described. The configuration of the inhaler device 100 is not limited to the above-described example. Various modifications illustrated below may be applied.

In an example, the number of the projections 143a provided at the bottom 143 of the container 140 is not limited to one and may be two or more. The number of the projections 145a provided at the inner wall 145 of the container 140 is not limited to eight and may be any number greater than or equal to one.

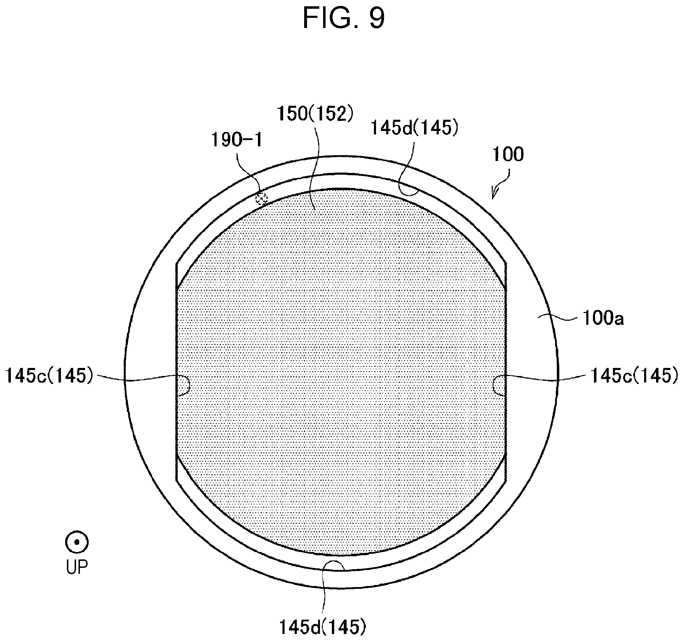

In another example, the shape of the inner wall 145 of the container 140 is not limited to the one having projections and recesses in the circumferential direction. This point will be described with reference to Fig. 9.

Fig. 9 is a view that illustrates an example of the configuration of the inner wall 145 of the container 140 according to the present embodiment. Fig. 9 schematically illustrates an example of a state where the inhaler device 100 and the stick substrate 150 inserted in the inhaler device 100 are viewed from above. As illustrated in Fig. 9, the inner wall 145 of the container 140 has a pair of opposite planar surfaces 145c. Furthermore, the inner wall 145 of the container 140 has a pair of facing circular arc curved surfaces 145d that connect both ends of the planar surfaces 145c. The pair of planar surfaces 145c and the pair of curved surfaces 145d are provided over the entire region in the up and down direction from the opening 142 to the bottom 143. In other words, the cross-sectional shape of the inner wall 145 in a plane orthogonal to the up and down direction is the same as the shape of the inner wall 145 when viewed from above, illustrated in Fig. 9. Thus, as illustrated in Fig. 9, the stick substrate 150 can be supported in a state where at least part of the side surface of the stick substrate 150 is spaced apart from the curved surfaces 145d. As a result, an air gap through which the airflow 190-1 passes can be formed between the side surface of the stick substrate 150 and the curved surfaces 145d. The curved surfaces 145d provided at the inner wall 145 are connected with the recesses 143b provided at the bottom 143. As a result, it is also possible to connect the airflow 190-1 with the airflow 190-2 without a hindrance. Furthermore, the width between the opposite planar surfaces 145c may be formed so as to be less than or equal to the width of the stick substrate 150. With the above configuration, the container 140 can sandwich the inserted stick substrate 150 while pressing the stick substrate 150 with the opposite planar surfaces 145c.

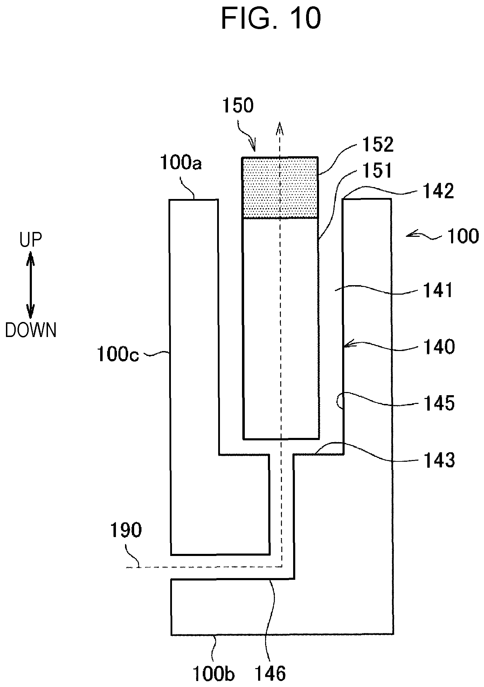

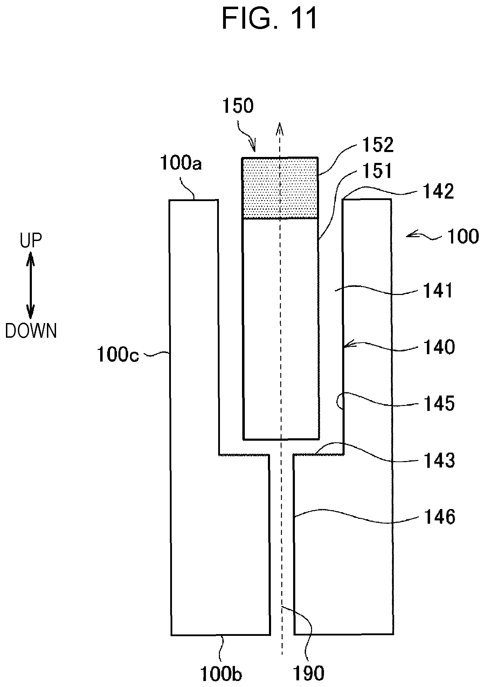

In another example, a mode of airflow that is generated in the inhaler device 100 as a result of puffing is not limited to counter flow. This point will be described with reference to Figs. 10 and 11.

Figs. 10 and 11 are views for illustrating examples of airflow that is generated in the inhaler device 100 as a result of puffing according to the present embodiment. Figs. 10 and 11 schematically illustrate examples of a cross section of the inhaler device 100 and the stick substrate 150 inserted in the inhaler device 100, taken in the up and down direction so as to pass through the center of the container 140. As illustrated in Fig. 10, the inhaler device 100 may have an airflow path 146 having an opening at the side surface 100c of the inhaler device 100 and an opening at the bottom 143 of the container 140. Then, airflow 190 may be generated so as to pass through the airflow path 146 as a result of puffing. As illustrated in Fig. 11, the inhaler device 100 may have an airflow path 146 having an opening at the bottom surface 100b of the inhaler device 100 and an opening at the bottom 143 of the container 140. Then, airflow 190 may be generated so as to pass through the airflow path 146 as a result of puffing. The opening of the airflow path 146 may be provided at the inner wall 145 of the container 140 instead of being provided at the bottom 143 of the container 140.

### 1.4. Trigger to start heating operation

The inhaler device 100 starts heating the stick substrate 150 in accordance with a predetermined trigger.

The inhaler device 100 may start heating in response to depression of the button 11 in a predetermined depression pattern as a trigger. A depression pattern is defined by the number of times the button 11 is depressed, the length of time during which the button 11 is depressed, and a rhythm with which the button 11 is depressed. For example, the inhaler device 100 may start heating operation in response to depression of the button 11 twice successively as a trigger or may start heating operation in response to depression and holding of the button 11 for a second as a trigger.

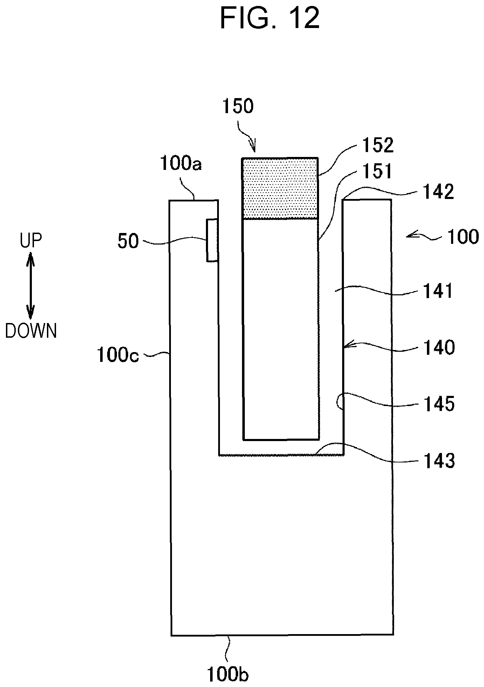

The inhaler device 100 may start heating operation in response to insertion of the stick substrate 150 as a trigger. Insertion and removal of the stick substrate 150 can be detected with a selected method. In an example, insertion and removal of the stick substrate 150 may be detected with a proximity sensor. The proximity sensor is a sensor that detects the approach of an object. Various methods can be adopted as a proximity sensor. Various methods include a method of detecting the approach of an object in accordance with a detection result of reflected waves obtained by radiating waves, such as ultrasonic waves and infrared rays, and a method of detecting the approach of an object in accordance with a change in capacitance. An example of arrangement of a proximity sensor will be described with reference to Fig. 12. Fig. 12 is a view for illustrating an example of arrangement of a proximity sensor in the inhaler device 100 according to the present embodiment. As illustrated in Fig. 12, a proximity sensor 50 may be disposed at the inner wall 145 of the container 140 near the opening 142. When the stick substrate 150 is inserted in the container 140, the proximity sensor 50 detects that an object is approaching. When the stick substrate 150 is removed from the container 140, the proximity sensor 50 detects that an object is not approaching. In this way, the proximity sensor 50 can detect insertion and removal of the stick substrate 150 by detecting the presence or absence of an approaching object.

A sensor that detects insertion and removal of the stick substrate 150 is not limited to the proximity sensor 50. A pressure sensor may be provided in addition to or instead of the proximity sensor 50. The pressure sensor can be provided in, for example, an area that contacts with the stick substrate 150 inserted, such as the projection 143a provided at the bottom 143 of the container 140, the projections 145a provided at the inner wall 145 of the container 140, and the planar surfaces 145c provided at the inner wall 145 of the container 140. Then, the pressure sensor may detect insertion and removal of the stick substrate 150 in accordance with the presence or absence of pressure.

The inhaler device 100 may start heating operation in response to connection of the charging device 900 as a trigger. Alternatively, the inhaler device 100 may start heating operation in response to the start of charging with the charging device 900 as a trigger.

The inhaler device 100 may start heating operation in response to release of connection with the charging device 900 as a trigger. Alternatively, the inhaler device 100 may start heating operation in response to the end of charging with the charging device 900 as a trigger. The inhaler device 100 may start heating operation in response to the fact that, as a result of charging with the charging device 900, the amount of electric power stored in the power supply 111 exceeds a predetermined threshold (for example, a switching threshold described below) as a trigger.

Which trigger is validated and which trigger is invalidated as a trigger to start heating operation may be set to be changeable. A trigger to start heating operation may be set by, for example, operation to the inhaler device 100, operation to the charging device 900, or remote operation via an external terminal, such as a smartphone.

Even when any trigger is validated, heating operation is desirably started on condition that the stick substrate 150 is inserted in the inhaler device 100. This is intended to prevent so-called no-substrate heating in which the heater 121 performs heating operation although no stick substrate 150 is inserted in the inhaler device 100.

### 1.5. Heating operation based on heating profile

The controller 116 controls the operation of the heater 121 in accordance with a heating profile. Controlling the operation of the heater 121 is implemented by controlling electric power supplied from the power supply 111 to the heater 121. The heater 121 heats the stick substrate 150 by using electric power supplied from the power supply 111.

A heating profile is control information for controlling a temperature at which an aerosol source is heated. A heating profile defines a parameter related to a temperature at which an aerosol source is heated. An example of the temperature at which an aerosol source is heated is the temperature of the heater 121. An example of a parameter related to the temperature at which an aerosol source is heated is a target value of the temperature (hereinafter, also referred to as target temperature) of the heater 121. The temperature of the heater 121 may be controlled so as to change according to an elapsed time from the start of heating operation. In this case, a heating profile includes information that defines time-series changes in target temperature. In another example, a heating profile can include a parameter that defines a method of supplying electric power to the heater 121 (hereinafter, also referred to as power supply parameter). A power supply parameter includes, for example, a voltage applied to the heater 121, an on or off state of electric power supplied to the heater 121, or a method of feedback control to be adopted.

The controller 116 controls the operation of the heater 121 such that the temperature (hereinafter, also referred to as real temperature) of the heater 121 changes similarly to the target temperature defined in a heating profile. The heating profile is typically designed such that a flavor to be tasted by a user is optimal when the user inhales an aerosol generated from the stick substrate 150. Thus, it is possible to optimize a flavor to be tasted by a user by controlling the operation of the heater 121 in accordance with the heating profile.

Temperature control of the heater 121 can be implemented by, for example, known feedback control. Feedback control may be, for example, proportional-integral-differential controller (PID control). The controller 116 can supply electric power from the power supply 111 to the heater 121 in a form of pulse based on pulse width modulation (PWM) or pulse frequency modulation (PFM). In this case, the controller 116 can execute temperature control of the heater 121 by adjusting a duty ratio or frequency of power pulse in feedback control. Alternatively, the controller 116 may execute simple on/off control in feedback control. For example, the controller 116 may perform heating operation with the heater 121 until the real temperature reaches a target temperature, suspend heating operation with the heater 121 in a case where the real temperature reaches the target temperature, and resume heating operation with the heater 121 when the real temperature becomes lower than the target temperature.

The temperature of the heater 121 can be quantified by, for example, measuring or estimating an electric resistance value of the heater 121 (more accurately, a heat element that is a component of the heater 121). This is because the electric resistance value of the heat element changes according to a temperature. The electric resistance value of the heat element can be estimated by, for example, measuring a decrease in voltage in the heat element. A decrease in voltage in the heat element can be measured with a voltage sensor that measures a potential difference applied to the heat element. In another example, the temperature of the heater 121 can be measured with a temperature sensor, such as a thermistor, installed around the heater 121.

A period from when a process of generating an aerosol by using the stick substrate 150 is started to when the process is ended is also referred to as heating session below. In other words, a heating session is a period during which the operation of the heater 121, that is, electric power supplied to the heater 121, is controlled in accordance with the heating profile. Commencement of a heating session is the timing at which heating operation based on the heating profile is started. Termination of a heating session is the timing at which generation of a sufficient amount of aerosol is stopped. A heating session includes a first-half preliminary heating period and a second-half puff available period. A puff available period is a period during which a sufficient amount of aerosol is assumed to be generated. A preliminary heating period is a period from when heating is started to when a puff available period is started. Heating operation performed in the preliminary heating period is also referred to as preliminary heating operation.

The notifier 113 may notify a user of information indicating the timing at which preliminary heating operation ends. For example, the notifier 113 notifies information notifying in advance the end of preliminary heating operation before preliminary heating operation ends or notifies information indicating that preliminary heating operation has ended at the timing at which preliminary heating operation ends. Notification to a user can be performed by, for example, turning on an LED, vibration, or the like. The user is able to take a puff just after the end of preliminary heating operation with the notification as a reference.

Similarly, the notifier 113 may notify a user of information indicating the timing at which the puff available period ends. For example, the notifier 113 notifies information notifying in advance the end of the puff available period before the puff available period ends or notifies information indicating that the puff available period has ended at the timing at which the puff available period ends. Notification to a user can be performed by, for example, turning on an LED, vibration, or the like. The user is able to take a puff until the end of the puff available period with the notification as a reference.

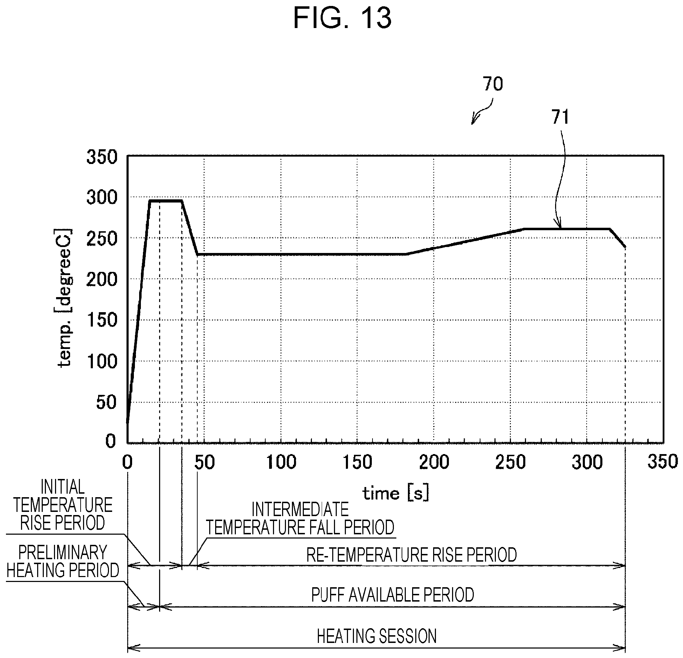

An example of the heating profile will be described with reference to Fig. 13. Fig. 13 is a graph that schematically illustrates an example of a heating profile according to the present embodiment. The abscissa axis of a graph 70 represents time. The ordinate axis of the graph 70 represents temperature. A line 71 represents time-series changes in target temperature. As illustrated in Fig. 13, a heating session may sequentially include an initial temperature rise period, an intermediate temperature fall period, and a re-temperature rise period.

The initial temperature rise period is an initial period of the heating session and is a period during which the temperature of the heater 121 increases from an initial temperature. The initial temperature is the temperature of the heater 121 at the start of heating operation. In the initial temperature rise period, the temperature of the heater 121 steeply increases and is maintained at a high temperature.

The intermediate temperature fall period is a period subsequent to the initial temperature rise period and is a period during which the temperature of the heater 121 decreases. The inhaler device 100 may suspend electric power supplied to the heater 121 in the intermediate temperature fall period.

The re-temperature rise period is a period subsequent to the intermediate temperature fall period and is a period during which the temperature of the heater 121 increases again. Typically, the rate of temperature rise of the heater 121 in the re-temperature rise period is set so as to be gentler than the rate of temperature rise of the heater 121 in the initial temperature rise period.

In the example illustrated in Fig. 13, the target temperature steeply increases to around 300°C in the initial temperature rise period, subsequently decreases to about 230°C in the intermediate temperature fall period, and then increases to around 260°C in the re-temperature rise period in a stepwise manner. In the example illustrated in Fig. 13, a period from the start of heating operation to the middle of the initial temperature rise period is the preliminary heating period, and a period from the middle of the initial temperature rise period to the termination of the re-temperature rise period is the puff available period.

### 1.6. Electric power supplied from charging device 900 to inhaler device 100

It can be difficult to continue heating operation until the end of the heating session even when the inhaler device 100 starts heating the stick substrate 150 in a state where the amount of electric power stored in the power supply 111 is low. When heating operation stops in the middle of the heating session, the quality of user experience remarkably degrades. Then, the aerosol generating system 1 executes a process described below to prevent such degradation of the quality of user experience.

The charging device 900 may charge the inhaler device 100 in a state where the inhaler device 100 and the charging device 900 are connected. In other words, the power supply 911 may charge the power supply 111 by supplying electric power to the power supply 111 in a state where the inhaler device 100 and the charging device 900 are connected.

When the inhaler device 100 performs heating operation in a state where the inhaler device 100 and the charging device 900 are connected, the charging device 900 may supply electric power for performing heating operation to the inhaler device 100. In other words, the power supply 911 may supply electric power to the heater 121 in a state where the inhaler device 100 and the charging device 900 are connected. Then, the heater 121 may heat the stick substrate 150 by using electric power supplied from the power supply 911.

When the inhaler device 100 performs heating operation in a state where the inhaler device 100 and the charging device 900 are connected, the charging device 900 may stop charging the inhaler device 100 and then supply electric power for performing heating operation to the inhaler device 100. Alternatively, when the inhaler device 100 performs heating operation in a state where the inhaler device 100 and the charging device 900 are connected, the charging device 900 may supply electric power for performing heating operation to the inhaler device 100 while charging the inhaler device 100. In other words, the power supply 911 can supply electric power to at least any one of the power supply 111 and the heater 121 in a state where the inhaler device 100 and the charging device 900 are connected.

However, when the heater 121 performs heating operation in a state where the inhaler device 100 and the charging device 900 are connected, the controller 116 selects a power supply source from the power supply 111 or the power supply 911 to the heater 121 in accordance with the amount of electric power stored in the inhaler device 100. In an example, when the amount of electric power stored in the power supply 111 is less than a threshold (hereinafter, also referred to as switching threshold), the controller 116 may select the power supply 911 as a power supply source to the heater 121. In another example, when the amount of electric power stored in the power supply 111 is greater than or equal to the switching threshold, the controller 116 may select the power supply 111 as a power supply source to the heater 121. The switching threshold corresponds to electric power at which heating operation can be continued until the end of the heating session. With the above configuration, when it is difficult to continue heating operation until the end of the heating session only with the amount of electric power stored in the power supply 111, the inhaler device 100 can continue heating operation until the end of the heating session by receiving electric power supplied from the power supply 911. Thus, it is possible to improve the quality of user experience.

When the power supply 911 is selected as a power supply source to the heater 121, the power supply 911 may supply electric power to only the heater 121. In other words, the charging device 900 may cause the inhaler device 100 to perform heating operation. When the power supply 911 is selected as a power supply source to the heater 121, the power supply 911 may supply electric power to both the heater 121 and the power supply 111. In other words, the charging device 900 may cause the inhaler device 100 to perform heating operation while charging the inhaler device 100.

The controller 116 may dynamically set the switching threshold. More specifically, the controller 116 may set different switching thresholds before heating operation and during heating operation. Furthermore, the controller 116 may change the switching threshold according to the progress of heating operation during heating operation. In an example, the controller 116 sets the switching threshold to a value at which heating operation can be continued from the beginning to the end of the heating session before heating operation based on the heating profile. In another example, the controller 116 sets the switching threshold to a value at which heating operation can be continued to the end of the remaining period of the heating session during heating operation based on the heating profile. In this case, the controller 116 reduces the switching threshold as heating operation progresses, that is, as the remaining time of the heating session, shortens. With the above configuration, it is possible to dynamically set the switching threshold depending on whether before heating operation or during heating operation and furthermore according to the progress of heating operation during heating operation. As a result, it is possible to further accurately determine whether it is possible to continue heating operation until the end of the heating session only with the amount of electric power stored in the power supply 111.

When the amount of electric power stored in the power supply 111 is less than the switching threshold, the controller 116 may select the power supply 911 as a power supply source to the heater 121 in at least part of the period of the heating session. With the above configuration, it is possible to prevent a decrease in electric power stored in the power supply 111 in at least part of the period of the heating session.

The at least part of the period of the heating session described above may include a period during which power consumption is relatively large (hereinafter, also referred to as high-load period). In other words, when the amount of electric power stored in the power supply 111 is less than the switching threshold, the inhaler device 100 may select the power supply 911 as a power supply source to the heater 121 in the high-load period. An example of the high-load period is a period including a period during which the temperature of the heater 121 increases and excluding a period during which the temperature of the heater 121 is maintained in the initial temperature rise period. Another example of the high-load period is the initial temperature rise period. Another example of the high-load period is the preliminary heating period. With the above configuration, when a power supply source to the heater 121 in the high-load period is set to the power supply 911, it is possible to effectively prevent a decrease in the amount of electric power stored in the power supply 111.

In a period other than the high-load period, the controller 116 may select the power supply 111 as a power supply source to the heater 121 when a predetermined condition is satisfied. Then, the controller 116 may select the power supply 911 as a power supply source to the heater 121 when the predetermined condition is not satisfied. In other words, the controller 116 may continuously select the power supply 911 as a power supply source to the heater 121 after the high-load period ends until the predetermined condition is satisfied, and switch the power supply source to the heater 121 to the power supply 111 when the predetermined condition is satisfied. With the above configuration, it is possible to continue heating operation until the end of the heating session while switching the power supply source to the heater 121 from the power supply 911 to the power supply 111 at appropriate timing.

The predetermined condition may include a condition that connection of the inhaler device 100 with the charging device 900 is released. In other words, the inhaler device 100 may set a power supply source to the heater 121 to the power supply 911 until connection of the inhaler device 100 with the charging device 900 is released and switch the power supply source to the power supply 111 after release. With the above configuration, even after connection of the inhaler device 100 with the charging device 900 is released, heating operation can be continued.

The predetermined condition may include a condition that the amount of electric power stored in the power supply 111 becomes greater than or equal to the switching threshold. In other words, the inhaler device 100 may set a power supply source to the heater 121 to the power supply 911 until the amount of electric power stored in the power supply 111 becomes greater than or equal to the switching threshold and switch the power supply source to the power supply 111 after the amount of electric power stored in the power supply 111 becomes greater than or equal to the switching threshold. With the above configuration, it is possible to continue heating operation based on electric power supplied from the power supply 911 until the amount of electric power stored in the power supply 111 becomes greater than or equal to the switching threshold.

The predetermined condition may include a condition that the high-load period ends. In other words, when the high-load period ends, the inhaler device 100 may automatically switch the power supply source to the heater 121 from the power supply 911 to the power supply 111.

The predetermined condition just needs to include at least any one of the illustrated conditions.

The notifier 113 or the notifier 913 may notify information indicating that connection of the inhaler device 100 with the charging device 900 is allowed to be released when the amount of electric power stored in the power supply 111 becomes greater than or equal to the switching threshold. A user is able to use the inhaler device 100 alone by releasing connection of the inhaler device 100 with the charging device 900 with the notification as a reference. With the above configuration, it is possible to further improve the quality of user experience.

The aerosol generating system 1 may operate such that the amount of electric power greater than or equal to the switching threshold is stored in the power supply 111 at the stage at which the high-load period ends.

In an example, when the heater 121 performs heating operation in a state where the inhaler device 100 and the charging device 900 are connected, the power supply 911 may increase a voltage applied to the power supply 111 as compared to a case where the heater 121 does not perform heating operation. With the above configuration, it is possible to rapidly charge the power supply 111 in a period before the high-load period ends. As a result, it is possible to store the amount of electric power greater than or equal to the switching threshold in the power supply 111 at the stage at which the high-load period ends.

In another example, the controller 116 may prohibit heating operation until the power supply 111 is charged with the amount of electric power greater than or equal to the switching threshold in a state where the inhaler device 100 and the charging device 900 are connected. Then, when the power supply 111 is charged with the amount of electric power greater than or equal to the switching threshold, the controller 116 may permit heating operation. With the above configuration, the inhaler device 100 can start heating operation only when the amount of electric power greater than or equal to the switching threshold is stored in the power supply 111.

When the aerosol generating system 1 operates as described above, the notifier 113 or the notifier 913 may notify information indicating that connection of the inhaler device 100 with the charging device 900 is allowed to be released in response to the end of the high-load period as a trigger. A user is able to use the inhaler device 100 alone by releasing connection of the inhaler device 100 with the charging device 900 with the notification as a reference. With the above configuration, it is possible to further improve the quality of user experience.

An example in which the aerosol generating system 1 operates such that the amount of electric power greater than or equal to the switching threshold is stored in the power supply 111 at the stage at which the high-load period ends has been described. The other supplementary note will be described below.

The power supply 911 may start supplying electric power to the heater 121 when the stick substrate 150 is inserted in the inhaler device 100 in a state where the inhaler device 100 and the charging device 900 are connected. Similarly, the power supply 911 may start supplying electric power to the heater 121 when the inhaler device 100 and the charging device 900 are connected in a state where the stick substrate 150 is inserted in the inhaler device 100. In other words, the power supply 911 may start supplying electric power to the heater 121 in response to fulfilment of two conditions, that is, connection of the inhaler device 100 with the charging device 900 and insertion of the stick substrate 150 in the inhaler device 100, as a trigger. With the above configuration, a user is able to start heating operation without, for example, depressing the button 91.

When the inhaler device 100 and the charging device 900 are connected, the power supply 911 may supply electric power to the power supply 111, may supply electric power to the heater 121, or may supply electric power to both the power supply 111 and the heater 121, as described above. In a state where the inhaler device 100 and the charging device 900 are connected, the controller 916 may set in accordance with a user operation to the charging device 900 whether the power supply 911 supplies electric power to the power supply 111, or supplies electric power to the heater 121, or supplies electric power to both the power supply 111 and the heater 121. For example, the charging device 900 may switch these settings when the button 91 is depressed in a predetermined depression pattern. With the above configuration, it is possible to implement supply of electric power as intended by a user.

When the inhaler device 100 and the charging device 900 are connected, the notifier 913 of the charging device 900 may notify the amount of electric power stored in the power supply 111 (that is, the progress of charging) and/or the progress of heating operation with the heater 121. On the other hand, when connection of the inhaler device 100 with the charging device 900 is released, the notifier 113 of the inhaler device 100 may notify the amount of electric power stored in the power supply 111 and/or the progress of heating operation with the heater 121.

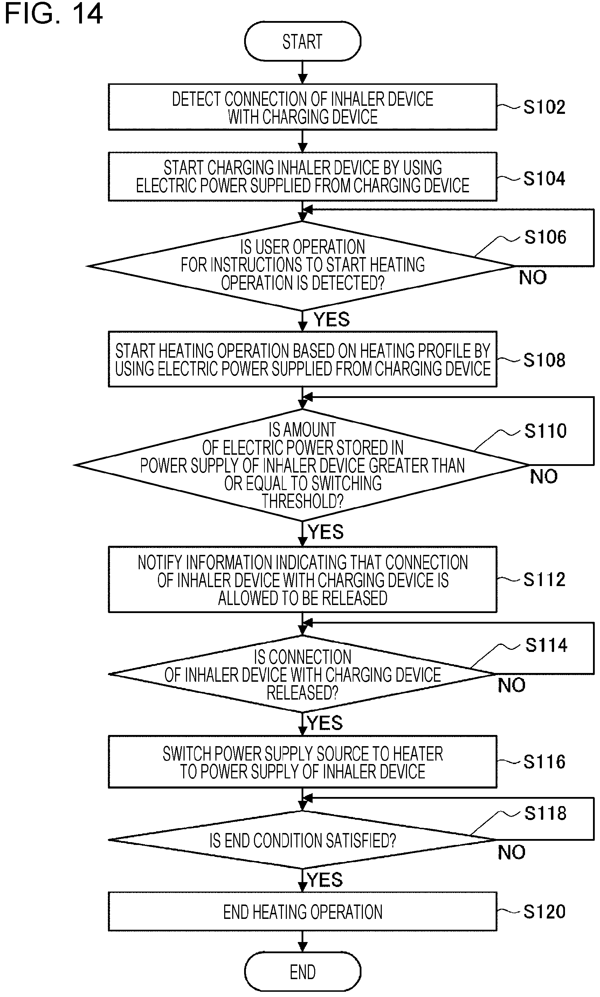

Hereinafter, an example of the flow of the above-described process will be described with reference to Fig. 14. Fig. 14 is a flowchart that illustrates an example of the flow of a process that is executed by the aerosol generating system 1 according to the present embodiment.

As illustrated in Fig. 14, initially, the controller 116 detects connection of the inhaler device 100 with the charging device 900 (step S102). For example, the controller 116 detects connection of the inhaler device 100 with the charging device 900 in accordance with the magnetic field of the magnetic portion 93, detected by the Hall sensor, and/or the presence or absence of electrical connection between the electrical contact 14 and the electrical contact 94.

Subsequently, the controller 116 starts charging the power supply 111 by using electric power supplied from the charging device 900 (step S104). In other words, the power supply 911 charges the power supply 111 by supplying electric power to the power supply 111.

Subsequently, the controller 116 determines whether a user operation for instructions to start heating operation is detected (step S106). An example of the user operation for instructions to start heating operation is depression of the button 91. Another example of the user operation for instructions to start heating operation is insertion of the stick substrate 150 in the inhaler device 100. The controller 116 waits until a user operation for instructions to start heating operation is detected (NO in step S106).

When a user operation for instructions to start heating operation is detected (YES in step S106), the controller 116 starts heating operation in accordance with the heating profile by using electric power supplied from the charging device 900 (step S108). In other words, the power supply 911 supplies electric power to the heater 121. Then, the heater 121 starts heating operation in accordance with the heating profile by using electric power supplied from the power supply 911.

Subsequently, the controller 116 determines whether the amount of electric power stored in the power supply 111 becomes greater than or equal to the switching threshold (step S110). The controller 116 waits until the amount of electric power stored in the power supply 111 becomes greater than or equal to the switching threshold (NO in step S110).

When it is determined that the amount of electric power stored in the power supply 111 becomes greater than or equal to the switching threshold (YES in step S110), the notifier 913 notifies information indicating that connection of the inhaler device 100 with the charging device 900 is allowed to be released (step S112). For example, the LED 92 emits light in a predetermined pattern of light.

Subsequently, the controller 116 determines whether connection of the inhaler device 100 with the charging device 900 is released (step S114). The controller 116 waits until connection of the inhaler device 100 with the charging device 900 is released (NO in step S114).

When it is determined that connection of the inhaler device 100 with the charging device 900 is released (YES in step S114), the controller 116 switches the power supply source to the heater 121 from the power supply 911 to the power supply 111 (step S116). In other words, the power supply 111 starts supplying electric power to the heater 121. Then, the heater 121 continues heating operation in accordance with the heating profile by using electric power supplied from the power supply 111.

Subsequently, the controller 116 determines whether an end condition is satisfied (step S118). An example of the end condition is that heating operation is performed until the end of the heating session. Another example of the end condition is that the number of puffs has reached a predetermined number of times. The controller 116 waits until the end condition is satisfied (NO in step S118).

When it is determined that the end condition is satisfied (YES in step S118), the controller 116 ends heating operation (step S120).

### 1.7. Switching of heating profile

The inhaler device 100 may switch the heating profile to be used at the time of heating the stick substrate 150. Hereinafter, in an example, an example in which the inhaler device 100 selects the heating profile to be used from between two heating profiles will be described. Of course, the inhaler device 100 may select the heating profile to be used from among three or more heating profiles.

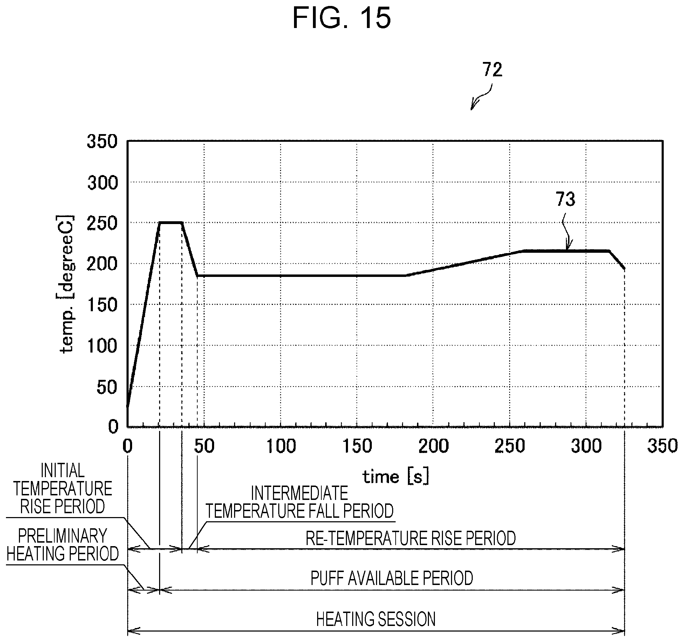

The controller 116 can select the heating profile to be used from between a first heating profile and a second heating profile. An example of the first heating profile is a heating profile illustrated in Fig. 13. An example of the second heating profile will be described with reference to Fig. 15.

Fig. 15 is a graph that schematically illustrates an example of a heating profile according to the present embodiment. The abscissa axis of a graph 72 represents time. The ordinate axis of the graph 72 represents temperature. A line 73 represents time-series changes in target temperature. As illustrated in Fig. 15, a heating session may sequentially include an initial temperature rise period, an intermediate temperature fall period, and a re-temperature rise period. In the example illustrated in Fig. 15, the target temperature steeply increases to around 250°C in the initial temperature rise period, subsequently decreases to about 180°C in the intermediate temperature fall period, and then increases to around 220°C in the re-temperature rise period in a stepwise manner. In the example illustrated in Fig. 15, a period from the start of heating operation to the middle of the initial temperature rise period is the preliminary heating period, and a period from the middle of the initial temperature rise period to the termination of the re-temperature rise period is the puff available period.

When the first heating profile illustrated in Fig. 13 is compared with the second heating profile illustrated in Fig. 15, the first heating profile is a high-temperature heating profile, and the second heating profile is a low-temperature heating profile. In this way, when the high-temperature heating profile and the low-temperature heating profile are allowed to be switched, a user is able to enjoy smoking feeling according to his or her feeling.

The controller 116 may select a heating profile to be used in accordance with a user operation to the inhaler device 100. In an example, depression patterns of the button 11 may be respectively associated with the first heating profile and the second heating profile. Then, the controller 116 may select the heating profile corresponding to a depression pattern at the time when the button 11 is depressed as a heating profile to be used. For example, the controller 116 may select the first heating profile as a heating profile to be used when the button 11 is depressed once or when the button 11 is depressed for a short period of time. Alternatively, the controller 116 may select the second heating profile as a heating profile to be used when the button 11 is depressed twice or when the button 11 is depressed for a long period of time. In another example, the controller 116 may switch the heating profile to be used from the first heating profile to the second heating profile or from the second heating profile to the first heating profile when the button 11 is depressed in a predetermined depression pattern.

The controller 116 may select the heating profile to be used in accordance with a user operation to the charging device 900. In an example, depression patterns of the button 91 may be respectively associated with the first heating profile and the second heating profile. Then, the controller 116 may select the heating profile corresponding to a depression pattern at the time when the button 91 is depressed as a heating profile to be used. For example, the controller 116 may select the first heating profile as a heating profile to be used when the button 91 is depressed once or when the button 91 is depressed for a short period of time. Alternatively, the controller 116 may select the second heating profile as a heating profile to be used when the button 91 is depressed twice or when the button 91 is depressed for a long period of time. In another example, the controller 116 may switch the heating profile to be used from the first heating profile to the second heating profile or from the second heating profile to the first heating profile when the button 91 is depressed in a predetermined depression pattern.

The controller 116 may select the heating profile to be used in accordance with a user operation to the inhaler device 100 when the inhaler device 100 and the charging device 900 are not connected. On the other hand, the controller 116 may select the heating profile to be used in accordance with a user operation to the charging device 900 when the inhaler device 100 and the charging device 900 are connected.

After a heating profile to be used is set, the inhaler device 100 starts heating operation based on the set heating profile in accordance with a selected trigger. A trigger to start heating operation is as described above. Alternatively, the inhaler device 100 may start heating operation in response to setting of a heating profile.

The controller 116 may set the heating profile to be used in a state where the inhaler device 100 and the charging device 900 are connected and the heating profile to be used in a state where the inhaler device 100 and the charging device 900 are not connected. For example, the controller 116 may use the first heating profile when the inhaler device 100 and the charging device 900 are connected and use the second heating profile when the inhaler device 100 and the charging device 900 are not connected.

The controller 116 may switch the heating profile to b used in accordance with a trigger to start heating operation. In an example, the controller 116 may use the first heating profile when heating is started in accordance with a user operation to the inhaler device 100 and use the second heating profile when heating is started in accordance with a user operation to the charging device 900. In another example, the controller 116 may use the first heating profile when heating operation is started in response to depression of the button 11 as a trigger and use the second heating profile when heating operation is used in response to insertion of the stick substrate 150 as a trigger.

Here, the controller 116 may switch the heating profile to be used during heating operation based on the heating profile. The controller 116 may switch the heating profile to be used when the above-described user operation is performed on the inhaler device 100 or the charging device 900 during heating operation based on the heating profile. In an example, the controller 116 may switch the heating profile to be used to the second heating profile during heating operation based on the first heating profile. Of course, it is also possible vice versa.

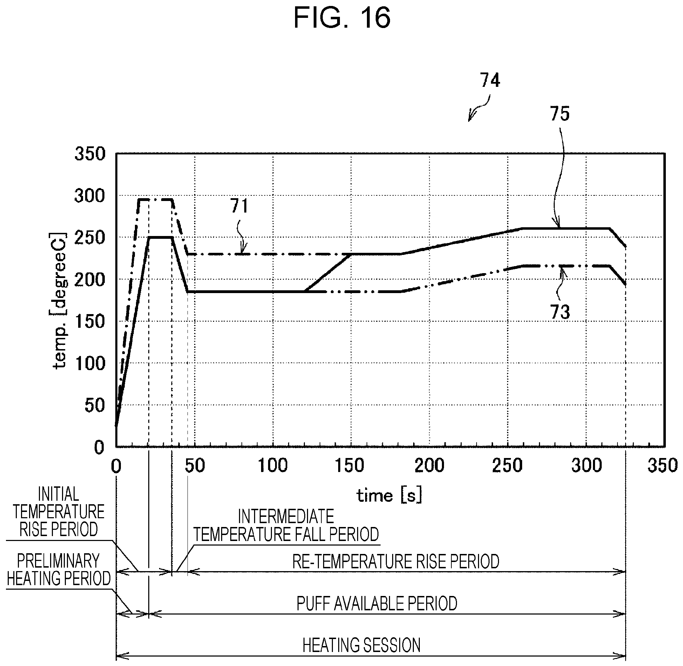

However, when the controller 116 switches the heating profile to be used, the controller 116 takes over a heating time before switching and controls the operation of the heater 121 by referring to the switched heating profile midway. For example, when the controller 116 switches the heating profile after a lapse of 120 seconds from the start of heating operation, the controller 116 controls the operation of the heater 121 in accordance with time-series changes in target temperature from the time after a lapse of 120 seconds from the start of heating operation in the switched heating profile. With the above configuration, it is possible to smoothly switch the heating profile in the middle of the heating session. In an example, an example in which the heating profile to be used is switched to the first heating profile during heating operation based on the second heating profile will be described with reference to Fig. 16.

Fig. 16 is a graph that schematically illustrates an example of switching of the heating profile according to the present embodiment. The abscissa axis of a graph 74 represents time. The ordinate axis of the graph 74 represents temperature. The line 71 represents time-series changes in target temperature, defined by the first heating profile. The line 73 represents time-series changes in target temperature, defined by the second heating profile. The line 75 represents time-series changes in target temperature when the heating profile to be used is switched to the first heating profile after a lapse of 120 seconds from the start of heating operation based on the second heating profile. As represented by the line 75, the target temperature changes as defined by the second heating profile until a lapse of 120 seconds from the start of heating operation. After that, the target temperature passes through a transitional period that smoothly fills a difference in target temperature between the second heating profile and the first heating profile and then changes as defined by the first heating profile.

### 1.8. Information notification

The charging device 900 (for example, the notifier 913) may notify information indicating the status of the inhaler device 100. Particularly, the notifier 913 of the charging device 900 may notify information indicating the status of the inhaler device 100 when the inhaler device 100 and the charging device 900 are connected. In an example, the LED 92 may notify information indicating the status of the inhaler device 100. In consideration of the fact that the LED 12 is hidden when the inhaler device 100 and the charging device 900 are connected, it is possible to improve usability with the above configuration.

The LED 92 notifies information indicating the status of the inhaler device 100 by emitting light in a pattern of light corresponding to the status of the inhaler device 100. A pattern of light is defined by at least one of the number, position, shape, and color of regions to emit light, the number of times of light emission, a light emission time, and a blinking rhythm.

### (1) Information indicating progress of heating operation

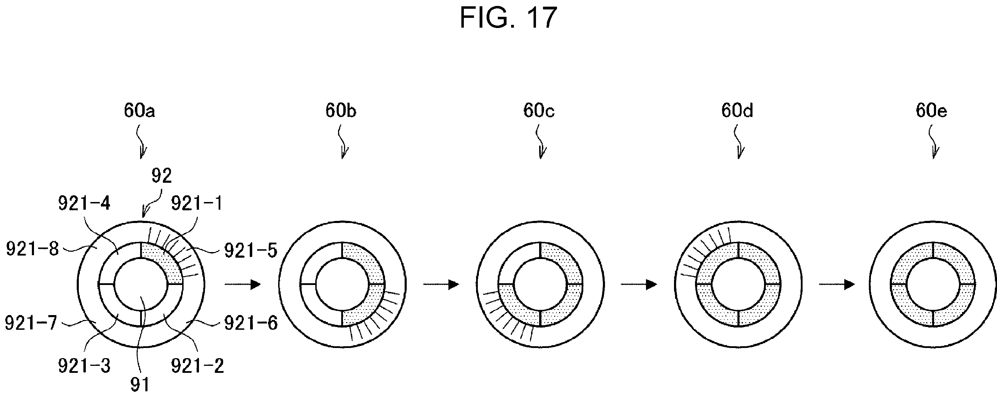

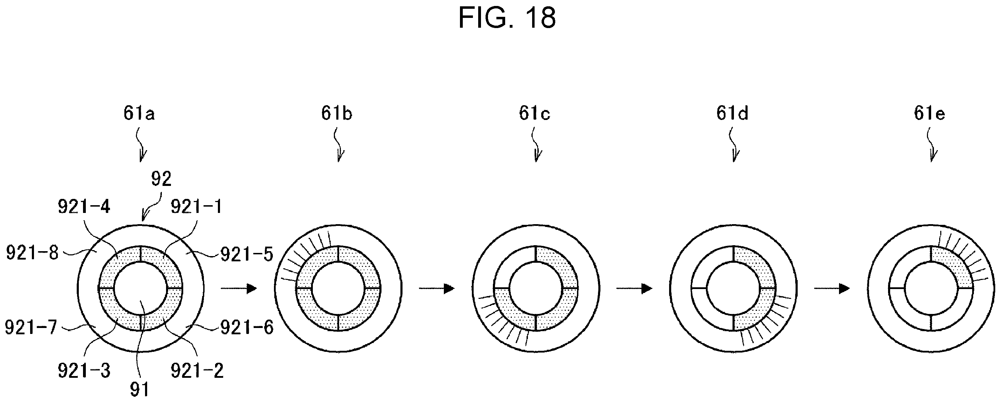

The notifier 913 may notify information indicating the progress of heating operation by the inhaler device 100 (more specifically, the heater 121). Particularly, the LED 92 may notify information indicating the progress of heating operation by the inhaler device 100. When heating operation is started in response to depression of the button 91 as a trigger, it is possible to intuitively notify a user of the progress of heating operation by notifying information indicating the progress of heating operation with the LED 92 disposed so as to surround the depressed button 91. An example of information indicating the progress of heating operation by the inhaler device 100, which is notified by the LED 92, will be described with reference to Figs. 17 and 18.

Fig. 17 is a view for illustrating an example of information that is notified by the LED 92 of the charging device 900 according to the present embodiment. Fig. 17 illustrates a state when the button 91 and the LED 92 are viewed from above. As illustrated in Fig. 17, the LED 92 may be formed in an annular shape so as to surround the circumference of the button 91 of which the top surface is formed in a circular shape. The LED 92 has a plurality of light emission regions 921 (921-1 to 921-8). The light emission regions 921-1 to 921-4 are circular arc regions obtained by dividing an inner region of the LED 92 formed in an annular shape into four every 90 degrees in the circumferential direction. The light emission regions 921-5 to 921-8 are circular arc regions respectively located on the outer sides of the light emission regions 921-1 to 921-4. When the light emission regions 921-1 to 921-4 emit light, the whole of each of the light emission regions 921-1 to 921-4 emits light. On the other hand, when the light emission regions 921-5 to 921-8 emit light, a plurality of lines extending radially about the button 91 emits light.

Fig. 17 illustrates patterns of light 60a to 60e. The pattern of light 60a is a pattern of light in which the light emission regions 921-1, 921-5 emit light. The pattern of light 60b is a pattern of light in which the light emission regions 921-1, 921-2, 921-6 emit light. The pattern of light 60c is a pattern of light in which the light emission regions 921-1 to 921-3, 921-7 emit light. The pattern of light 60d is a pattern of light in which the light emission regions 921-1 to 921-4, 921-8 emit light. The pattern of light 60e is a pattern of light in which the light emission regions 921-1 to 921-4 emit light.

The LED 92 may emit light while switching a pattern of light sequentially from the pattern of light 60a to the pattern of light 60e according to the progress of heating operation by the inhaler device 100 in a period just after the start of heating operation. In an example, it is assumed that the length of the preliminary heating period is 20 seconds. In this case, the LED 92 may emit light in the pattern of light 60a for five seconds after the start of heating operation, in the pattern of light 60b for five seconds after that, in the pattern of light 60c for five seconds after that, and in the pattern of light 60d for five seconds after that. After a lapse of a period of 20 seconds after the start of heating operation, which is the preliminary heating period, the LED 92 may emit light in the pattern of light 60e. A user is able to easily grasp the remaining time of the preliminary heating period by referencing such changes in pattern of light.

Fig. 18 is a view for illustrating an example of information that is notified by the LED 92 of the charging device 900 according to the present embodiment. Fig. 18 illustrates a state when the button 91 and the LED 92 are viewed from above. The configurations of the button 91 and the LED 92 illustrated in Fig. 18 are as described above with reference to Fig. 17.

Fig. 18 illustrates patterns of light 61a to 61e. The pattern of light 61a is similar to the pattern of light 60e. The pattern of light 61b is similar to the pattern of light 60d. The pattern of light 61c is similar to the pattern of light 60c. The pattern of light 61d is similar to the pattern of light 60b. The pattern of light 61e is similar to the pattern of light 60a.

The LED 92 may emit light while switching a pattern of light sequentially from the pattern of light 61a to the pattern of light 61e according to the progress of heating operation by the inhaler device 100 in a period just before the end of heating operation. In an example, the LED 92 may emit light in the pattern of light 61a from the end of the preliminary heating period to 20 seconds before the end of heating operation. The LED 92 may emit light in the pattern of light 61b for five seconds from 20 seconds before the end of heating operation, in the pattern of light 61c for five seconds after that, in the pattern of light 61d for five seconds after that, and in the pattern of light 61e for five seconds after that. A user is able to easily grasp the remaining time to the end of heating operation by referencing such changes in pattern of light.

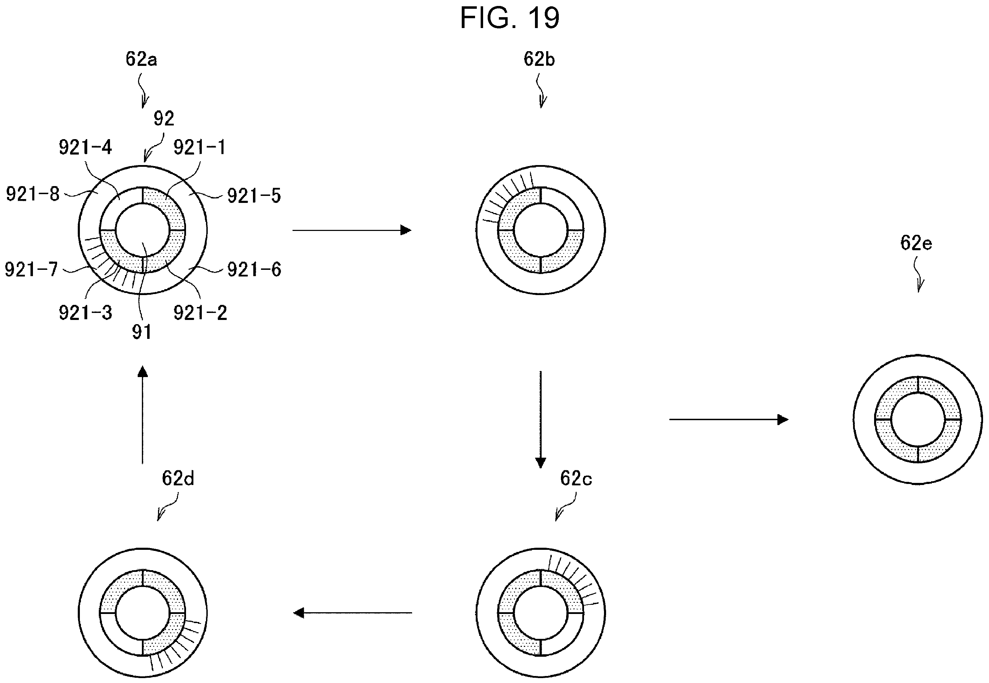

An example of information indicating the progress of heating operation by the inhaler device 100, which is notified by the LED 92, has been described with reference to Figs. 17 and 18. Subsequently, another example of information indicating the progress of heating operation by the inhaler device 100, which is notified by the LED 92, will be described with reference to Fig. 19.

Fig. 19 is a view for illustrating an example of information that is notified by the LED 92 of the charging device 900 according to the present embodiment. Fig. 19 illustrates a state when the button 91 and the LED 92 are viewed from above. The configurations of the button 91 and the LED 92 illustrated in Fig. 19 are as described above with reference to Fig. 17.

Fig. 19 illustrates patterns of light 62a to 62e. The pattern of light 62a is a pattern of light in which the light emission regions 921-1 to 921-3, 7 emit light. The pattern of light 62b is a pattern of light in which the light emission regions 921-2 to 921-4, 921-8 emit light. The pattern of light 62c is a pattern of light in which the light emission regions 921-1, 921-3, 921-4, 921-5 emit light. The pattern of light 62d is a pattern of light in which the light emission regions 921-1, 921-2, 921-4, 921-6 emit light. The pattern of light 62e is a pattern of light in which the light emission regions 921-1 to 921-4 emit light.

The LED 92 may emit light while repeatedly switching a pattern of light sequentially from the pattern of light 62a to the pattern of light 62d in the preliminary heating period. Then, the LED 92 may emit light in the pattern of light 62e after the end of preliminary heating period. A user is able to easily grasp whether the preliminary heating period is continuing or the preliminary heating period has ended by referencing such changes in pattern of light.

An example of information indicating the progress of heating operation by the inhaler device 100, which is notified by the LED 92, has been described with reference to Fig. 19.

Of course, information indicating the progress of heating operation by the inhaler device 100 may be notified with a method other than the positions of the light emission regions 921 that emit light in the LED 92. Other than the above, the LED 92 may notify information indicating the progress of heating operation by the inhaler device 100 with luminescent color of the LED 92. For example, the LED 92 may switch the luminescent color in order of red, yellow, and blue according to the progress of heating operation by the inhaler device 100 in a period just after the start of heating operation. Then, the LED 92 may switch the luminescent color in order of blue, yellow, and red according to the progress of heating operation by the inhaler device 100 in a period just before the end of heating operation.

As described above, the inhaler device 100 can switch the heating profile to be used at the time of heating the stick substrate 150. Then, the LED 92 may emit light in a pattern of light according to a heating profile used by the inhaler device 100. For example, the LED 92 may vary the luminescent color, and/or the positions of the light emission regions 921 between when the inhaler device 100 uses the first heating profile and when the inhaler device 100 uses the second heating profile. In an example, the LED 92 may notify information indicating the progress of heating operation by the inhaler device 100, described above with reference to Figs. 17 and 18, when the inhaler device 100 uses the first heating profile. On the other hand, the LED 92 may notify information indicating the progress of heating operation by the inhaler device 100, described above with reference to Fig. 19, when the inhaler device 100 uses the second heating profile. A user is able to easily grasp which heating profile is used by referencing such notification.

### (2) Information indicating state of charge

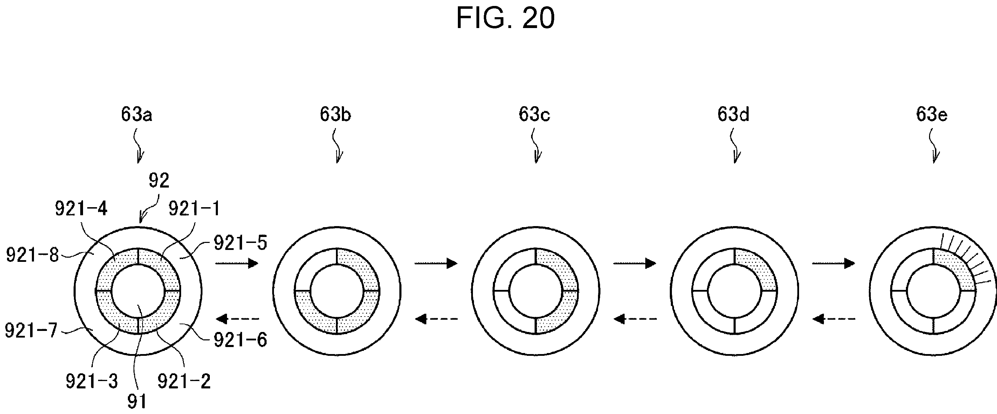

The notifier 913 may notify information indicating the state of charge of the inhaler device 100 (more specifically, the power supply 111). In other words, the notifier 913 may notify information indicating electric power stored in the power supply 111. Particularly, the LED 92 may notify information indicating the state of charge of the inhaler device 100. An example of information indicating the state of charge of the inhaler device 100, which is notified by the LED 92, will be described with reference to Fig. 20.

Fig. 20 is a view for illustrating an example of information that is notified by the LED 92 of the charging device 900 according to the present embodiment. Fig. 20 illustrates a state when the button 91 and the LED 92 are viewed from above. The configurations of the button 91 and the LED 92 illustrated in Fig. 20 are as described above with reference to Fig. 17.

Fig. 20 illustrates patterns of light 63a to 63e. The pattern of light 63a is a pattern of light in which the light emission regions 921-1 to 921-4 emit light. The pattern of light 63b is a pattern of light in which the light emission regions 921-1 to 921-3 emit light. The pattern of light 63c is a pattern of light in which the light emission regions 921-1, 921-2 emit light. The pattern of light 63d is a pattern of light in which the light emission region 921-1 emits light. The pattern of light 63e is a pattern of light in which the light emission regions 921-1, 921-5 emit light.

The LED 92 emits light in any one of the patterns of light 63a to 63e according to the amount of electric power stored in the power supply 111. In an example, the LED 92 may emit light in the pattern of light 63a when the amount of electric power stored in the power supply 111 ranges from 80% to 100% of a full state of charge, in the pattern of light 63b when the amount of electric power ranges from 60% to 80%, and in the pattern of light 63c when the amount of electric power ranges from 40% to 60%. The LED 92 may emit light in the pattern of light 63d when the amount of electric power stored in the power supply 111 ranges from 20% to 40% of the full state of charge, and in the pattern of light 63e when the amount of electric power ranges from 0% to 20%. In this case, the LED 92 emits light while switching the pattern of light sequentially from the pattern of light 63a to the pattern of light 63e with a decrease in the amount of electric power stored in the power supply 111. On the other hand, the LED 92 emits light while switching the pattern of light sequentially from the pattern of light 63e to the pattern of light 63a with an increase in the amount of electric power stored in the power supply 111 through charging. A user is able to easily grasp whether charging is needed or the progress of charging by referencing such notification.

Of course, information indicating the state of charge of the inhaler device 100 may be notified with a method other than the positions of the light emission regions 921 that emit light in the LED 92. Other than the above, the LED 92 may notify information indicating the state of charge of the inhaler device 100 with luminescent color of the LED 92. For example, the LED 92 may emit light in blue when the amount of electric power stored in the power supply 111 ranges from 50% to 100% of the full state of charge, in yellow when the amount of electric power ranges from 20% to 50%, and in red when the amount of electric power ranges from 0% to 20%.

The LED 92 may notify information indicating the number of times that heating operation is available (hereinafter, also referred to as a remaining number of times of heating operation) in accordance with a heating profile with the amount of electric power stored in the power supply 111 as information indicating the state of charge of the inhaler device 100. In an example, the LED 92 may emit light in the pattern of light 63a when the remaining number of times of heating operation is greater than or equal to 20, in the pattern of light 63b when the remaining number of times ranges from 15 to 19, and in the pattern of light 63c when the remaining number of times ranges from 10 to 14. The LED 92 may emit light in the pattern of light 63d when the remaining number of times of heating operation ranges from five to nine, and in the pattern of light 63e when the remaining number of times is less than or equal to four.

The charging device 900 may notify information indicating the state of charge of the charging device 900. In other words, the charging device 900 may notify information indicating the amount of electric power stored in the power supply 911. Information indicating the state of charge of the charging device 900 may be notified with a similar method to information indicating the state of charge of the inhaler device 100.

### (3) Error

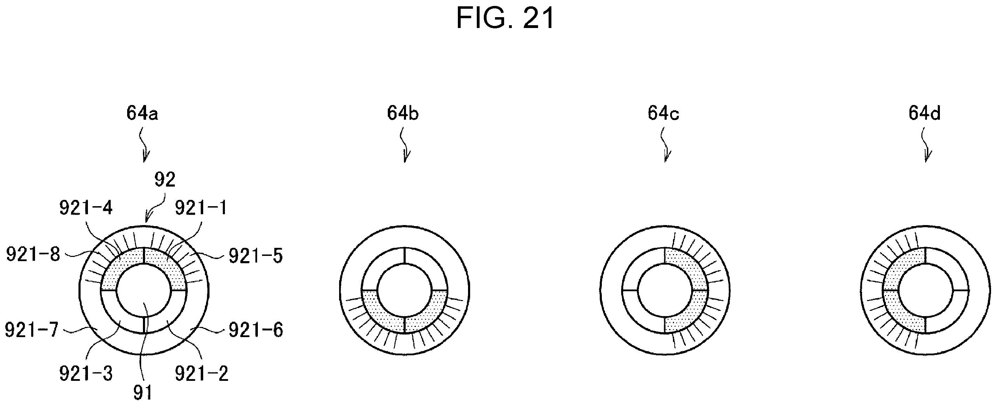

The notifier 913 may notify information indicating an error that has occurred in the inhaler device 100. Particularly, the LED 92 may notify information indicating an error that has occurred in the inhaler device 100. An example of information indicating an error that has occurred in the inhaler device 100, which is notified by the LED 92, will be described with reference to Fig. 21.

Fig. 21 is a view for illustrating an example of information that is notified by the LED 92 of the charging device 900 according to the present embodiment. Fig. 21 illustrates a state when the button 91 and the LED 92 are viewed from above. The configurations of the button 91 and the LED 92 illustrated in Fig. 21 are as described above with reference to Fig. 17.

Fig. 21 illustrates patterns of light 64a to 64d. The pattern of light 64a is a pattern of light in which the light emission regions 921-1, 921-4, 921-5, 921-8 emit light. The pattern of light 64b is a pattern of light in which the light emission regions 921-2, 921-3, 921-6, 921-7 emit light. The pattern of light 64c is a pattern of light in which the light emission regions 921-1, 921-2, 921-5, 921-6 emit light. The pattern of light 64d is a pattern of light in which the light emission regions 921-3, 921-4, 921-7, 921-8 emit light.

The LED 92 may emit light in the pattern of light 64a when a first error has occurred in the inhaler device 100, in the pattern of light 64b when a second error has occurred, in the pattern of light 64c when a third error has occurred, and in the pattern of light 64d when a fourth error has occurred. An example of the first error is that the temperature of the power supply 111 is extremely low. An example of the second error is that the temperature of the power supply 111 is extremely high. An example of the third error is that the temperature of the heater 121 is extremely low. An example of the fourth error is that the temperature of the heater 121 is extremely high.

Of course, information indicating an error that has occurred in the inhaler device 100 may be notified with a method other than the positions of the light emission regions 921 that emit light in the LED 92. Other than the above, the LED 92 may notify information indicating an error that has occurred in the inhaler device 100 with luminescent color of the LED 92. For example, the LED 92 may emit light in color according to an error that has occurred in the inhaler device 100.

The charging device 900 may notify information indicating an error that has occurred in the charging device 900. Information indicating an error that has occurred in the charging device 900 may be notified with a similar method to information indicating an error that has occurred in the inhaler device 100.

### (4) Notification timing

Various triggers to notify information indicating the status of the inhaler device 100 are conceivable.

In an example, information indicating the progress of heating operation by the inhaler device 100 may be notified during heating operation by the inhaler device 100. Information indicating the state of charge of the inhaler device 100 may be notified during charging of the inhaler device 100. When the inhaler device 100 and the charging device 900 are connected while there is an error in the inhaler device 100, information indicating an error that has occurred in the inhaler device 100 may be notified.

In another example, information indicating the status of the inhaler device 100 may be notified in response to depression of the button 91 as a trigger. However, different information may be notified according to a depression pattern of the button 91. Specifically, when the button 91 is depressed in a first depression pattern, information indicating the progress of heating operation by the inhaler device 100 may be notified. When the button 91 is depressed in a second depression pattern, information indicating the state of charge of the inhaler device 100 may be notified. When the button 91 is depressed in a third depression pattern, information indicating an error that has occurred in the inhaler device 100 may be notified. The first depression pattern, the second depression pattern, and the third depression pattern are different from one another. However, the first depression pattern may be the same as a depression pattern for instructions to start heating operation.

In another example, information indicating the status of the inhaler device 100 may be notified in response to connection of the inhaler device 100 with the charging device 900 or releasing connection of the inhaler device 100 with the charging device 900 as a trigger. Other than the above, information indicating the status of the inhaler device 100 may be notified in response to turning-off of the power, turning-on of the power, sleeping, or return from sleep of the inhaler device 100 as a trigger.

### (5) Supplement

Information indicating the status of the inhaler device 100 may be notified by the inhaler device 100 instead of the charging device 900 or together with the charging device 900. Particularly, the notifier 113 of the inhaler device 100 may notify information indicating the status of the inhaler device 100 when the inhaler device 100 and the charging device 900 are not connected. In an example, the LED 12 may notify information indicating the status of the inhaler device 100. The LED 12 can notify information indicating the status of the inhaler device 100 with a method similar to the method in which the LED 92 notifies information indicating the status of the inhaler device 100, described above.

The inhaler device 100 may notify information indicating the status of the charging device 900. Particularly, the inhaler device 100 may notify information indicating the status of the charging device 900 when the inhaler device 100 is connected to the charging device 900. In an example, the LED 12 may notify information indicating the status of the charging device 900. Examples of the information indicating the status of the charging device 900, which is notified by the inhaler device 100, include information indicating the state of charge of the charging device 900 and information indicating an error that has occurred in the charging device 900.

### 2. Modifications

An embodiment of the present disclosure has been described. Hereinafter, various modifications of the present disclosure will be described.

### 2.1. First modification

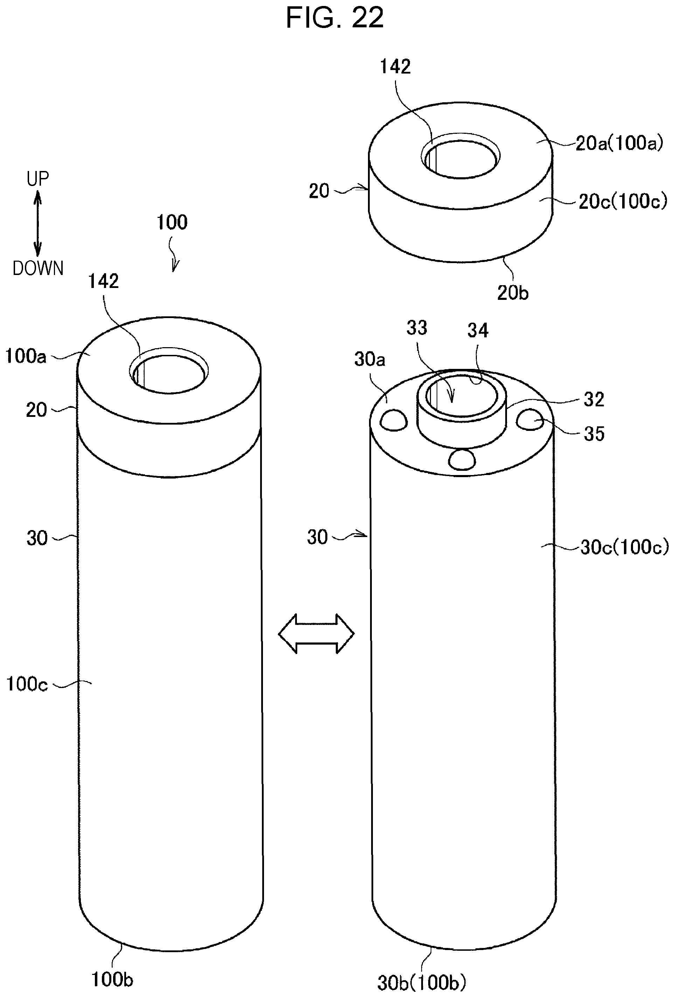

As described above, the cap 20 and the body 30 may be configured to be detachable. Various attachment and detachment mechanisms for the cap 20 and the body 30 are conceivable. Hereinafter, an example of an attachment and detachment mechanism for the cap 20 and the body 30 will be described with reference to Figs. 22 to 29.

### (1) First example

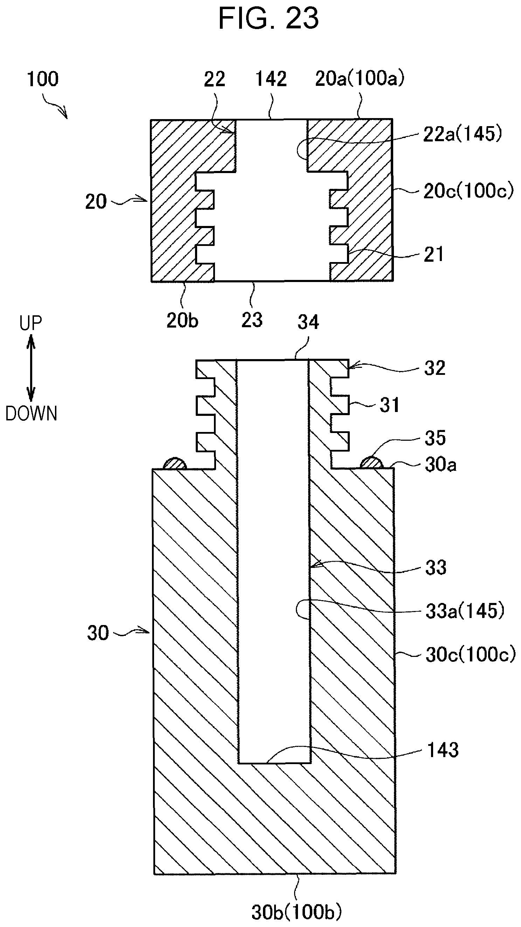

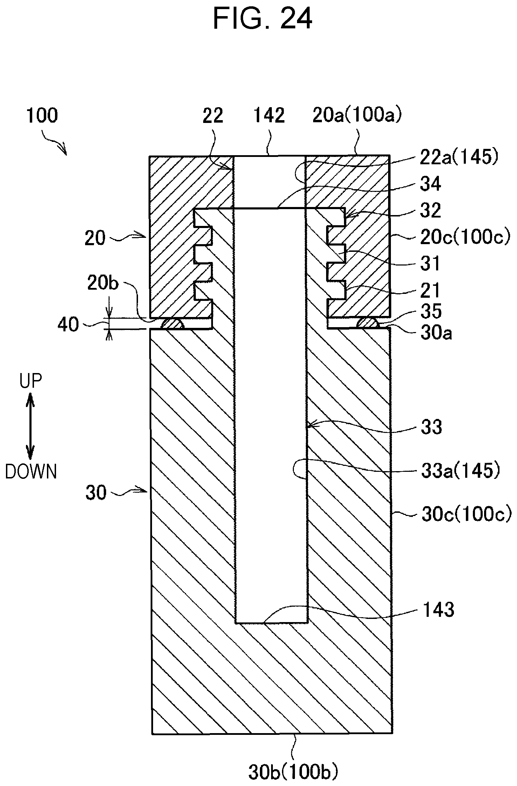

Fig. 22 is a view for illustrating a first example of the attachment and detachment mechanism of the inhaler device 100 according to the present modification. Fig. 23 is a view that schematically illustrates an example of a cross section of the inhaler device 100 in a state where connection of the cap 20 with the body 30 is released in the first example of the attachment and detachment mechanism. Fig. 24 is a view that schematically illustrates an example of a cross section of the inhaler device 100 in a state where the cap 20 and the body 30 are connected in the first example of the attachment and detachment mechanism. Figs. 23 and 24 schematically illustrate examples of a cross section of the inhaler device 100, taken in the up and down direction so as to pass through the center of the container 140.

As illustrated in Figs. 22 to 24, the cap 20 and the body 30 are connected such that a bottom surface 20b of the cap 20 and a top surface 30a of the body 30 overlap each other. The top surface 20a of the cap 20 is a component of the top surface 100a of the inhaler device 100. The bottom surface 30b of the body 30 is a component of the bottom surface 100b of the inhaler device 100. In a state where the cap 20 and the body 30 are connected, a side surface 20c of the cap 20 and a side surface 30c of the body 30 are components of the side surface 100c of the inhaler device 100.

As illustrated in Figs. 22 to 24, the cap 20 is formed as an annular body having a through-hole 22 with the opening 142 and an opening 23 at both ends. A thread groove 21 that can be screwed to a screw thread 31 provided at the body 30 is provided at a lower side of an inner wall 22a of the through-hole 22 of the cap 20. The body 30 is formed as a closed-end cylindrical body having a closed-end hole 33 with an opening 34 and the bottom 143 at both ends. The body 30 has the opening 34 at the top surface at the center of the top surface 30a, has the screw thread 31 on the outer periphery, and has a cylindrical protrusion 32 extending along the closed-end hole 33. The cap 20 and the body 30 can be connected or connection can be released by rotating the cap 20 and the body 30 in a state where the screw thread 31 is meshed with the thread groove 21. In the inhaler device 100 in a state where the cap 20 and the body 30 are connected, an air gap 40 can be provided between the bottom surface 20b of the cap 20 and the top surface 30a of the body 30.

As illustrated in Figs. 23 and 24, an upper side (a part where no thread groove 21 is provided) of the inner wall 22a of the through-hole 22 of the cap 20 is a component of an upper part of the inner wall 145 of the container 140. On the other hand, an inner wall 33a of the closed-end hole 33 of the body 30 is a component of a lower part of the inner wall 145 of the container 140. Then, in a state where the cap 20 and the body 30 are connected, the through-hole 22 and the closed-end hole 33 are connected and make up the container 140. Particularly, the upper side of the inner wall 22a of the through-hole 22 of the cap 20 and the inner wall 33a of the closed-end hole 33 of the body 30 are connected so as to be flush with each other and make up the inner wall 145 of the container 140.

Here, the projections 145a and the recesses 145b described with reference to Figs. 6 to 8 are desirably provided at both the upper side of the inner wall 22a of the through-hole 22 of the cap 20 and the inner wall 33a of the closed-end hole 33 of the body 30. Similarly, the planar surfaces 145c and the curved surfaces 145d described with reference to Fig. 9 are desirably provided at both the upper side of the inner wall 22a of the through-hole 22 of the cap 20 and the inner wall 33a of the closed-end hole 33 of the body 30.

As illustrated in Figs. 22 to 24, a plurality of nonslip pads 35 is provided around the protrusion 32 on the top surface 30a of the body 30. In the inhaler device 100 in a state where the cap 20 and the body 30 are connected, the nonslip pads 35 prevent rotation of the cap 20 by contacting with the bottom surface 20b of the cap 20. With the above configuration, it is possible to make it difficult to unintentionally release connection of the cap 20 with the body 30. The configuration of the nonslip pads 35 is not limited and may be made up of, for example, an elastic body, such as rubber. The nonslip pads 35 may be provided at the bottom surface 20b of the cap 20 together with or instead of the top surface 30a of the body 30.

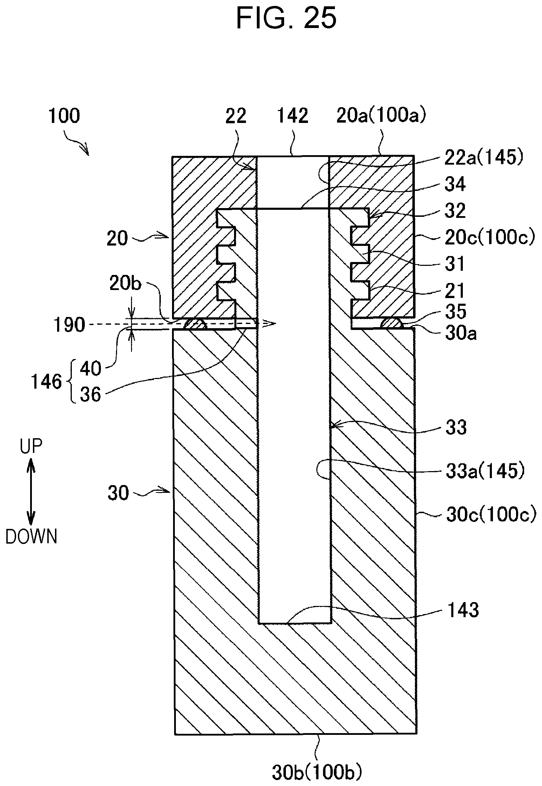

As described with reference to Figs. 10 to 11, the inhaler device 100 may have the airflow path 146. This point will be described with reference to Fig. 25.

Fig. 25 is a view that schematically illustrates another example of a cross section of the inhaler device 100 in a state where the cap 20 and the body 30 are connected in the first example of the attachment and detachment mechanism. As illustrated in Fig. 25, a through-hole 36 may be provided at a proximal part of the protrusion 32 of the body 30. In this case, in a state where the cap 20 and the body 30 are connected, an air gap 40 provided between the bottom surface 20b of the cap 20 and the top surface 30a of the body 30 and the through-hole 36 make up the airflow path 146. In this case, airflow 190 may be generated so as to pass through the airflow path 146 as a result of puffing.

### (2) Second example

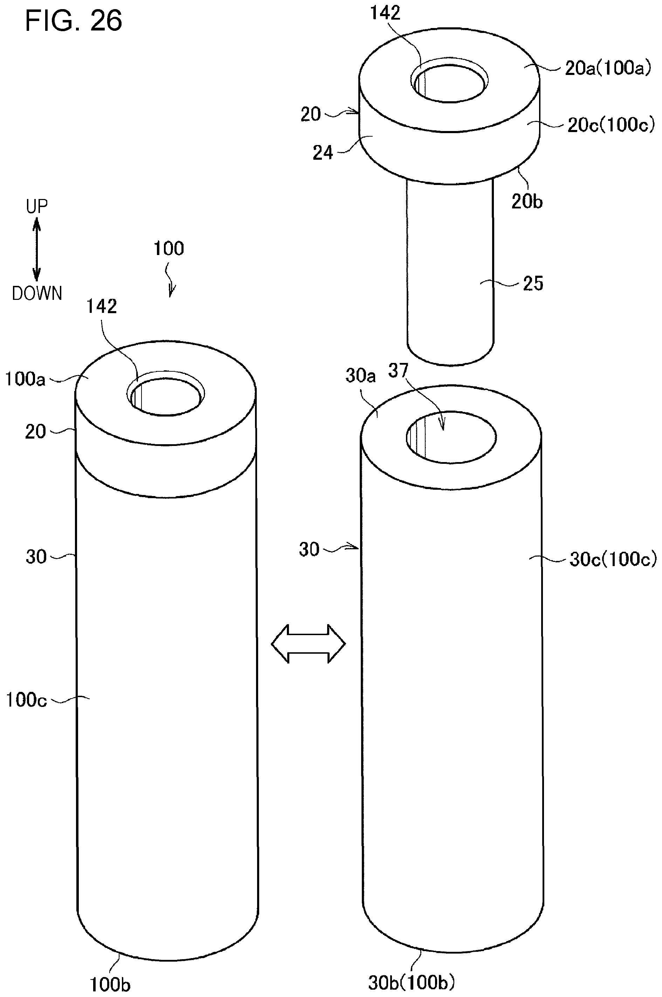

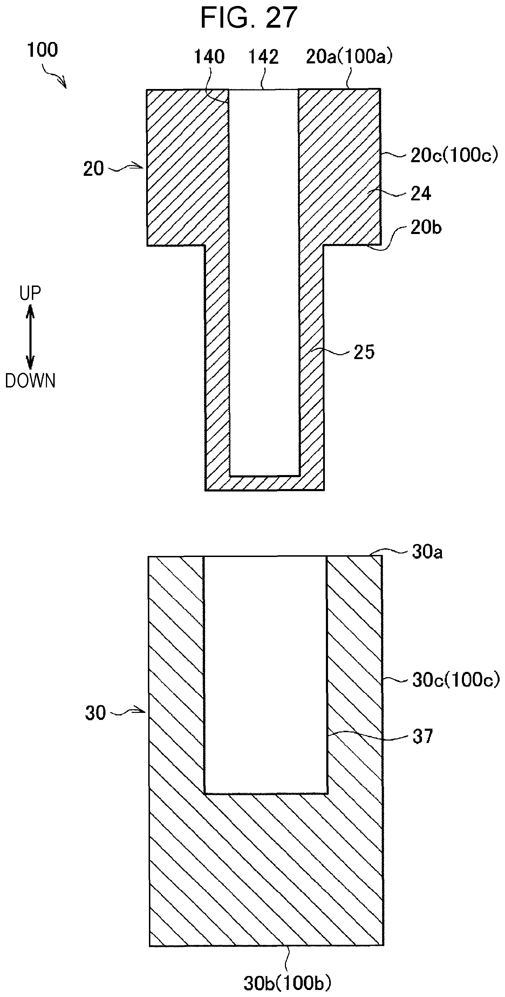

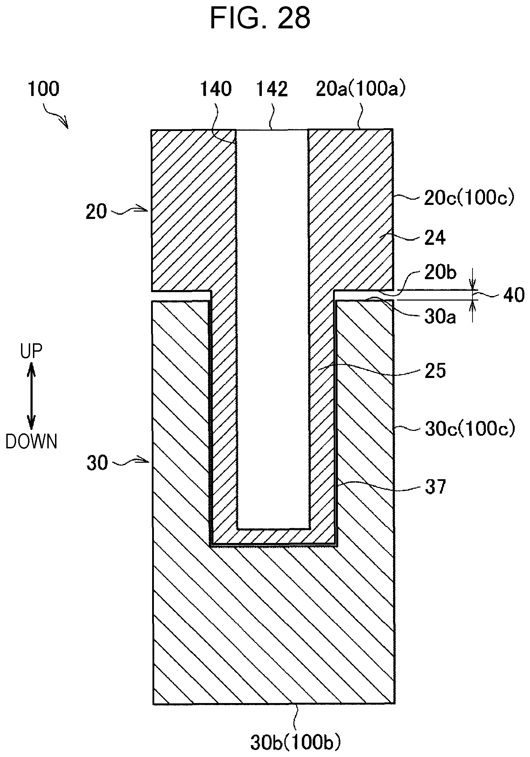

Fig. 26 is a view for illustrating a second example of the attachment and detachment mechanism of the inhaler device 100 according to the present modification. Fig. 27 is a view that schematically illustrates an example of a cross section of the inhaler device 100 in a state where connection of the cap 20 with the body 30 is released in the second example of the attachment and detachment mechanism. Fig. 28 is a view that schematically illustrates an example of a cross section of the inhaler device 100 in a state where the cap 20 and the body 30 are connected in the second example of the attachment and detachment mechanism. Figs. 27 and 28 schematically illustrate examples of a cross section of the inhaler device 100, taken in the up and down direction so as to pass through the center of the container 140.

As illustrated in Figs. 26 to 28, the cap 20 and the body 30 are connected such that the bottom surface 20b of the cap 20 and the top surface 30a of the body 30 overlap each other. The top surface 20a of the cap 20 is a component of the top surface 100a of the inhaler device 100. The bottom surface 30b of the body 30 is a component of the bottom surface 100b of the inhaler device 100. In a state where the cap 20 and the body 30 are connected, the side surface 20c of the cap 20 and the side surface 30c of the body 30 are components of the side surface 100c of the inhaler device 100.

As illustrated in Figs. 26 to 28, the cap 20 is a closed-end cylindrical body having the container 140. The cap 20 has a head 24 that is a component of the upper side of the cap 20 and a protrusion 25 formed so as to be thinner than the head 24 and protruding downward from the head 24. The body 30 has a closed-end hole 37 capable of accommodating the protrusion 25 of the cap 20. As illustrated in Figs. 27 and 28, the cap 20 and the body 30 can be connected by inserting the protrusion 25 of the cap 20 to the closed-end hole 37 of the body 30. Connection of the cap 20 with the body 30 can be released by removing the protrusion 25 of the cap 20 from the closed-end hole 37 of the body 30. Since the container 140 can be removed from the body 30 in which electronic components including the power supply 111 and the like are stored, it is possible to easily wash the container 140 with water, so it is possible to improve maintainability. In the inhaler device 100 in a state where the cap 20 and the body 30 are connected, the air gap 40 can be provided between the bottom surface 20b of the cap 20 (more specifically, the head 24 of the cap 20) and the top surface 30a of the body 30.

As illustrated in Figs. 27 and 28, the inside diameter of the closed-end hole 37 is desirably the same or substantially the same as the outside diameter of the protrusion 25 of the cap 20. In this case, in a state where the cap 20 and the body 30 are connected, it is possible to bring the protrusion 25 of the cap 20 and the closed-end hole 37 of the body 30 into close contact with each other without any gap, and firmly connect the cap 20 with the body 30. As illustrated in Figs. 27 and 28, the outside diameter of the head 24 of the cap 20 is desirably the same as the outside diameter of the body 30. In this case, in a state where the cap 20 and the body 30 are connected, it is possible to reduce unevenness between the side surface 20c of the cap 20 and the side surface 30c of the body 30 to smooth the side surface 100c of the inhaler device 100.

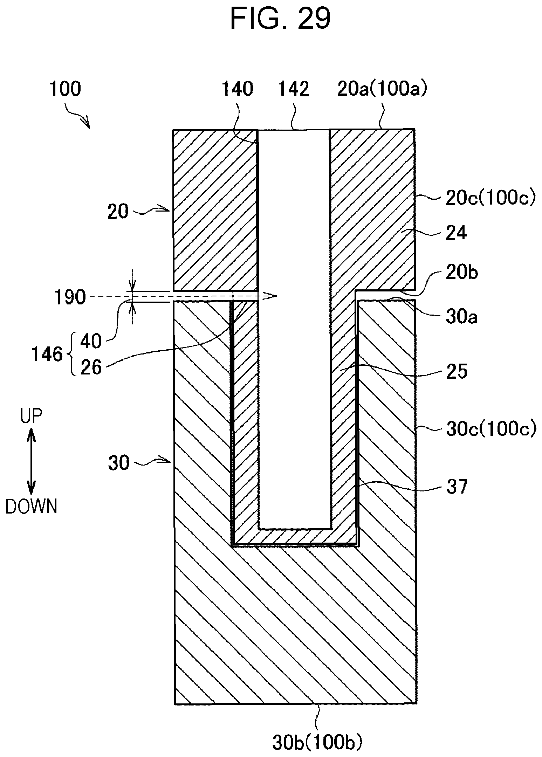

As described with reference to Figs. 10 to 11, the inhaler device 100 may have the airflow path 146. This point will be described with reference to Fig. 29.

Fig. 29 is a view that schematically illustrates another example of a cross section of the inhaler device 100 in a state where the cap 20 and the body 30 are connected in the second example of the attachment and detachment mechanism. As illustrated in Fig. 29, a through-hole 26 may be provided at a proximal part of the protrusion 25 of the cap 20. In this case, in a state where the cap 20 and the body 30 are connected, an air gap 40 provided between the bottom surface 20b of the cap 20 and the top surface 30a of the body 30 and the through-hole 26 can make up the airflow path 146. In this case, airflow 190 may be generated so as to pass through the airflow path 146 as a result of puffing.

### (3) Control according to attachment of cap 20 to body 30 or detachment of cap 20 from body 30

Connection of the cap 20 to the body 30 and release of connection can be determined with a selected method. For example, the cap 20 may have a magnetic portion that generates a magnetic field. Then, the body 30 may have a magnetic sensor that detects a magnetic field. An example of the magnetic portion is a magnet. An example of the magnetic sensor is a Hall sensor. The controller 116 determines whether the cap 20 and the body 30 are connected, in accordance with a detection result of the magnetic sensor. For example, the controller 116 determines that the cap 20 and the body 30 are connected when the strength of magnetic field detected by the magnetic sensor is greater than or equal to a threshold; otherwise, the controller 116 determines that connection of the cap 20 with the body 30 is released. With the above configuration, it is possible to easily determine connection of the cap 20 to the body 30 and release of connection.

The inhaler device 100 may perform setting based on whether the cap 20 and the body 30 are connected. In an example, the controller 116 may set whether to perform heating operation with the heater 121 in accordance with whether the cap 20 and the body 30 are connected. In other words, the controller 116 may permit heating operation with the heater 121 when the cap 20 and the body 30 are connected. When a user operation for instructions to start heating operation is performed in a state where heating operation is permitted, the controller 116 starts supplying electric power to the heater 121. On the other hand, the controller 116 may prohibit heating operation with the heater 121 when connection of the cap 20 with the body 30 is released. Even when a user operation for instruction to start heating operation is performed in a state where heating operation is prohibited, the controller 116 does not start supplying electric power to the heater 121. In a state where the cap 20 is removed from the body 30, the heat insulating properties of the inhaler device 100 can decrease. In terms of this point, with the above configuration, it is possible to improve safety.

The type of the cap 20 connected to the body 30 can be identified with a selected method. For example, the controller 116 may determine the type of the cap 20 connected to the body 30 in accordance with a detection result of the magnetic sensor. In an example, the cap 20 may have a magnetic portion that generates a magnetic field that varies by the type of the cap 20. Specifically, the cap 20 may have a magnetic portion that generates a magnetic field having a strength that varies by the type of the cap 20. In this case, the controller 116 may determine that the cap 20 of a first type is connected to the body 30 when the strength of a magnetic field detected by the magnetic sensor is greater than or equal to a first threshold and less than a second threshold. On the other hand, the controller 116 may determine that the cap 20 of a second type is connected to the body 30 when the strength of a magnetic field detected by the magnetic sensor is greater than or equal to the second threshold and less than a third threshold. With the above configuration, it is possible to easily determine the type of the cap 20 connected to the body 30.

The inhaler device 100 may perform setting based on the type of the cap 20 connected to the body 30. In an example, the controller 116 may set a heating profile used to heat the stick substrate 150 in accordance with the type of the cap 20 connected to the body 30. More specifically, the controller 116 may perform setting such that the first heating profile is used when the cap 20 of the first type is connected to the body 30. The controller 116 may perform setting such that the second heating profile is used when the cap 20 of the second type is connected to the body 30. The first heating profile is a heating profile different from the second heating profile. In another example, the controller 116 may set a notification method of the notifier 113 in accordance with the type of the cap 20 connected to the body 30. More specifically, the controller 116 may set a first pattern of light as a pattern of light of the LED 12 and/or the LED 92 when the cap 20 of the first type is connected to the body 30. The controller 116 may set a second pattern of light as a pattern of light of the LED 12 and/or the LED 92 when the cap 20 of the second type is connected to the body 30. The second pattern of light is a pattern of light different from the first pattern of light. With the above configuration, it is possible to easily customize the use comfort of the inhaler device 100.

Other than the above, the cap 20 may have a storage medium that stores setting information of the inhaler device 100. An example of the setting information is a heating profile. Another example of the setting information is a notification method of the notifier 113. On the other hand, the body 30 may have a reader that reads information from the storage medium of the cap 20 attached to the body 30. An example of the storage medium is an RF tag of radio frequency identifier (RFID). An example of the reader is a wireless reader. Then, the controller 116 may perform setting based on setting information read from the storage medium of the cap 20 connected to the body 30.

### 2.2. Second modification

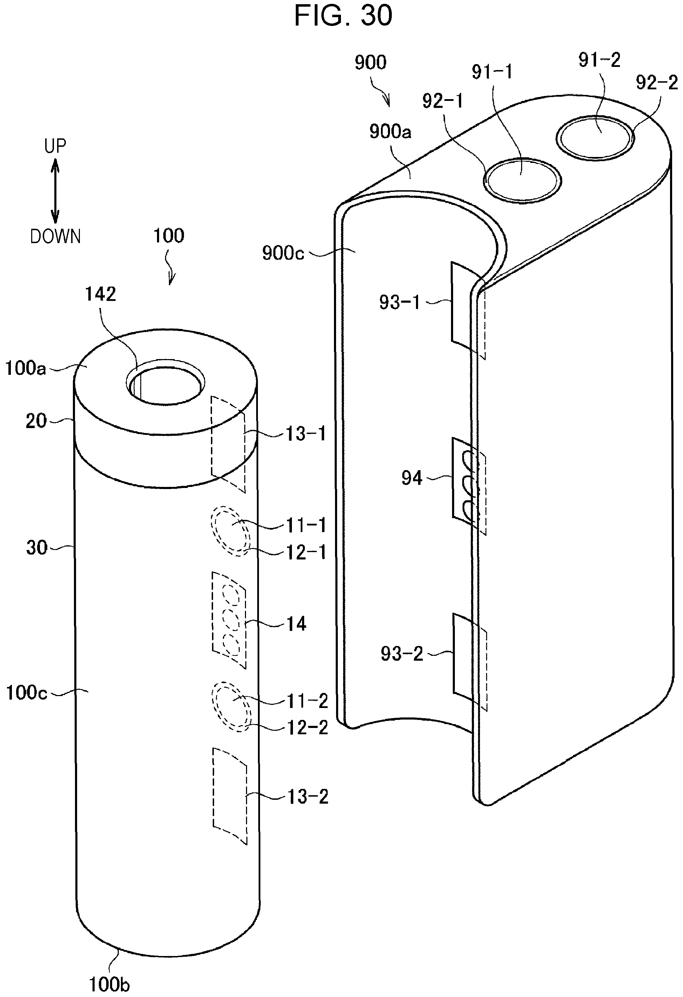

Fig. 30 is a view for illustrating the outline of the aerosol generating system 1 according to the modification. Particularly, Fig. 30 illustrates an example of a state where connection of the inhaler device 100 with the charging device 900 is released according to the present modification.

### (1) Button 11 and LED 12

As illustrated in Fig. 30, a button 11-1 and a button 11-2 are disposed in the inhaler device 100. The button 11-1 is an example of a first operating portion capable of receiving a user operation to the aerosol generating system 1. The button 11-2 is an example of a second operating portion different from the first operating portion and capable of receiving a user operation to the aerosol generating system 1.

The controller 116 may control the operation of the heater 121 in accordance with a heating profile corresponding to the button 11-1 when the button 11-1 is depressed. For example, the memory 114 stores the first heating profile as the heating profile to be used when the button 11-1 is depressed. In this case, the controller 116 controls the operation of the heater 121 in accordance with the first heating profile when the button 11-1 is depressed. On the other hand, the controller 116 may control the operation of the heater 121 in accordance with a heating profile corresponding to the button 11-2 when the button 11-2 is depressed. For example, the memory 114 stores the second heating profile as the heating profile to be used when the button 11-2 is depressed. In this case, the controller 116 controls the operation of the heater 121 in accordance with the second heating profile when the button 11-2 is depressed. With the above configuration, a user is able to use a favorite heating profile by depressing the button 11-1 or the button 11-2.

Here, the inhaler device 100 may switch the heating profile to be used during heating operation based on the heating profile. However, when the controller 116 switches the heating profile to be used, the controller 116 takes over a heating time before switching and controls the operation of the heater 121 by referencing the switched heating profile midway. More specifically, when the button 11-2 is depressed while the controller 116 is controlling the operation of the heater 121 in accordance with the heating profile corresponding to the button 11-1, the controller 116 controls the operation of the heater 121 by referencing the heating profile corresponding to the button 11-2 midway. For example, it is assumed that heating operation based on the first heating profile is started in response to depression of the button 11-1 as a trigger and then the button 11-2 is depressed after a lapse of 120 seconds. In this case, the controller 116 controls the operation of the heater 121 in accordance with time-series changes in target temperature from the time after a lapse of 120 seconds from the start of heating operation in the second heating profile. Similarly, when the button 11-1 is depressed while the controller 116 is controlling the operation of the heater 121 in accordance with the heating profile corresponding to the button 11-2, the controller 116 controls the operation of the heater 121 by referencing the heating profile corresponding to the button 11-1 midway. For example, it is assumed that heating operation based on the second heating profile is started in response to depression of the button 11-2 as a trigger and then the button 11-1 is depressed after a lapse of 120 seconds. In this case, the controller 116 controls the operation of the heater 121 in accordance with time-series changes in target temperature from the time after a lapse of 120 seconds from the start of heating operation in the first heating profile. With the above configuration, it is possible to smoothly switch the heating profile in the middle of the heating session.

As illustrated in Fig. 30, an LED 12-1 and an LED 12-2 are disposed in the inhaler device 100. The LED 12-1 and the LED 12-2 are examples of the notifier that outputs information to be notified to a user from the aerosol generating system 1. Particularly, the LED 12-1 is an example of a first notifier disposed in association with the button 11-1. Specifically, the LED 12-1 is disposed so as to surround the button 11-1. The LED 12-2 is an example of a second notifier disposed in association with the button 11-2. Specifically, the LED 12-2 is disposed so as to surround the button 11-2.

The LED 12-1 may notify information on a process executed in response to depression of the button 11-1 as a trigger. In an example, the LED 12-1 may notify information indicating the progress of heating operation based on the heating profile corresponding to the button 11-1. More specifically, the controller 116 may control the operation of the heater 121 in accordance with the first heating profile when the button 11-1 is depressed and notify through the LED 12-1 information indicating the progress of heating operation based on the first heating profile. Similarly, the LED 12-2 may notify information on a process executed in response to depression of the button 11-2 as a trigger. In an example, the LED 12-2 may notify information indicating the progress of heating operation based on the heating profile corresponding to the button 11-2. More specifically, the controller 116 may control the operation of the heater 121 in accordance with the second heating profile when the button 11-2 is depressed and notify through the LED 12-2 information indicating the progress of heating operation based on the second heating profile. With the above configuration, it is possible to further clarify the relationship between user operation and notification.

The LED 12-1 and the LED 12-2 may notify information indicating the state of charge of the inhaler device 100. Particularly, the LED 12-1 may notify information indicating the remaining number of times of heating operation when the heating profile corresponding to the button 11-1 is used. For example, when the heating profile corresponding to the button 11-2 is the first heating profile, the remaining number of times of heating operation when the heating profile corresponding to the button 11-1 is used is the number of times that heating operation based on the first heating profile can be performed with the amount of electric power stored in the power supply 111. On the other hand, the LED 12-2 may notify information indicating the remaining number of times of heating operation when the heating profile corresponding to the button 11-2 is used. For example, when the heating profile corresponding to the button 11-2 is the second heating profile, the remaining number of times of heating operation when the heating profile corresponding to the button 11-2 is used is the number of times that heating operation based on the second heating profile can be performed with the amount of electric power stored in the power supply 111. The amount of electric power that is consumed at the time of heating operation can vary between the heating profile corresponding to the button 11-1 and the heating profile corresponding to the button 11-2. For example, the high-temperature heating profile as in the case of the first heating profile illustrated in Fig. 13 consumes a larger amount of electric power at the time of heating operation than the low-temperature heating profile as in the case of the second heating profile illustrated in Fig. 15. For this reason, the remaining number of times of heating operation when the first heating profile is used can be less than the remaining number of times of heating operation when the second heating profile is used. With the above configuration, the remaining number of times of heating operation, which varies depending on the heating profile to be used, can be notified for each heating profile. Therefore, it is possible to improve usability related to selection of a heating profile to be used, such as selecting a heating profile of which the remaining number of times of heating operation is greater.

Notification of the remaining number of times of heating operation when the heating profile corresponding to the button 11-1 is used and notification of the remaining number of times of heating operation when the heating profile corresponding to the button 11-2 is used may be performed at different timings. For example, the LED 12-1 may notify the remaining number of times of heating operation when the heating profile corresponding to the button 11-1 is used in a case where the button 11-1 is depressed. The LED 12-2 may notify the remaining number of times of heating operation when the heating profile corresponding to the button 11-2 is used in a case where the button 11-2 is depressed.

Of course, notification of the remaining number of times of heating operation when the heating profile corresponding to the button 11-1 is used and notification of the remaining number of times of heating operation when the heating profile corresponding to the button 11-2 is used may be performed at the same time. For example, these notifications may be performed at the same time in response to turning-off of the power, turning-on of the power, sleeping, or return from sleep of the inhaler device 100 as a trigger.

As described in the first modification, the cap 20 and the body 30 may be configured to be detachable in the present modification as well. Then, the inhaler device 100 may perform setting based on the type of the cap 20 connected to the body 30.

In an example, the inhaler device 100 may set a heating profile used to heat the stick substrate 150 in accordance with the type of the cap 20 connected to the body 30. More specifically, the controller 116 may set at least any one of the heating profile corresponding to the button 11-1 and the heating profile corresponding to the button 11-2 in accordance with the type of the cap 20 connected to the body 30. For example, the controller 116 may associate the first heating profile with the button 11-1 and associate the second heating profile with the button 11-2 when the cap 20 of the first type is connected to the body 30. The controller 116 may associate the second heating profile with the button 11-1 and associate the first heating profile with the button 11-2 when the cap 20 of the second type is connected to the body 30. With the above configuration, it is possible to easily customize the use comfort of the inhaler device 100.

In another example, the inhaler device 100 may set a notification method of the notifier 113 in accordance with the type of the cap 20 connected to the body 30. More specifically, the controller 116 may set a notification method with at least any one of the LED 12-1 and the LED 12-2 in accordance with the type of the cap 20 connected to the body 30. For example, the controller 116 may set a first pattern of light as a pattern of light of the LED 12-1 and set a second pattern of light as a pattern of light of the LED 12-2 when the cap 20 of the first type is connected to the body 30. The controller 116 may set a second pattern of light as a pattern of light of the LED 12-1 and set a first pattern of light as a pattern of light of the LED 12-2 when the cap 20 of the second type is connected to the body 30. With the above configuration, it is possible to easily customize the use comfort of the inhaler device 100.

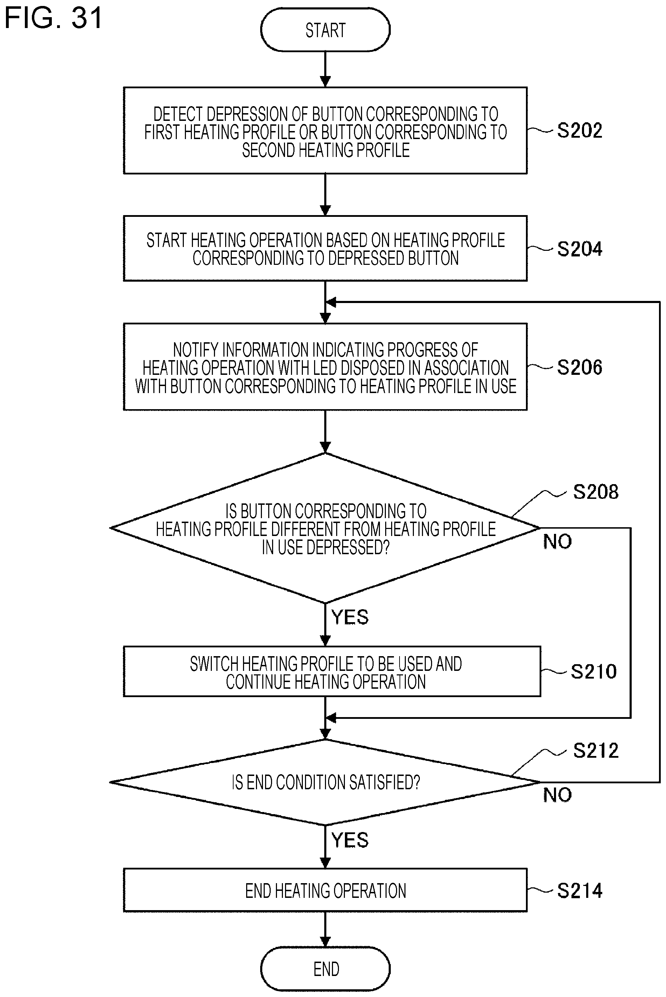

Next, an example of the flow of a process that is executed in the present modification will be described with reference to Fig. 31. Fig. 31 is a flowchart that illustrates an example of the flow of a process that is executed by the inhaler device 100 according to the present modification. Hereinafter, it is assumed that the first heating profile is associated with the button 11-1 and the second heating profile is associated with the button 11-2.

As illustrated in Fig. 31, initially, the sensor 112 detects depression of the button 11-1 corresponding to the first heating profile or the button 11-2 corresponding to the second heating profile (step S202).

Subsequently, the controller 116 starts heating operation based on the heating profile corresponding to the depressed button 11 (step S204). In an example, the controller 116 starts controlling the operation of the heater 121 in accordance with the first heating profile when the button 11-1 is depressed. In another example, the controller 116 starts controlling the operation of the heater 121 in accordance with the second heating profile when the button 11-2 is depressed.

Subsequently, the controller 116 causes the LED 12 disposed in association with the button 11 corresponding to the heating profile in use to notify information indicating the progress of heating operation (step S206). In an example, when the first heating profile is in use, the LED 12-1 disposed in association with the button 11-1 corresponding to the first heating profile notifies information indicating the progress of heating operation based on the first heating profile. In another example, when the second heating profile is in use, the LED 12-2 disposed in association with the button 11-2 corresponding to the second heating profile notifies information indicating the progress of heating operation based on the second heating profile.

Subsequently, the controller 116 determines whether the button 11 corresponding to the heating profile different from the heating profile in use is depressed (step S208). In an example, the controller 116 determines whether the button 11-2 is depressed during heating operation based on the first heating profile. In another example, the controller 116 determines whether the button 11-1 is depressed during heating operation based on the second heating profile.

When it is determined that the button 11 corresponding to the heating profile different from the heating profile in use is depressed (YES in step S208), the controller 116 switches the heating profile to be used and continues heating operation (step S210). For example, when the controller 116 switches the heating profile after a lapse of 120 seconds from the start of heating operation, the controller 116 controls the operation of the heater 121 in accordance with time-series changes in target temperature from the time after a lapse of 120 seconds from the start of heating operation in the switched heating profile. After that, the process proceeds to step S212. When it is determined that the button 11 corresponding to the heating profile different from the heating profile in use is not depressed (NO in step S208) as well, the process proceeds to step S212.

In step S212, the controller 116 determines whether the end condition is satisfied (step S212). An example of the end condition is that heating operation is performed until the end of the heating session. Another example of the end condition is that the number of puffs has reached a predetermined number of times. When it is determined that the end condition is not satisfied (NO in step S212), the process returns to step S206.

When it is determined that the end condition is satisfied (YES in step S212), the controller 116 ends heating operation (step S214).

### (2) Button 91 and LED 92

By referring to Fig. 30 again, a button 91-1 and a button 91-2 are disposed in the charging device 900. The button 91-1 is an example of a first operating portion capable of receiving a user operation to the aerosol generating system 1. The button 91-2 is an example of a second operating portion different from the first operating portion and capable of receiving a user operation to the aerosol generating system 1.

By referring to Fig. 30 again, an LED 92-2 and an LED 92-2 are disposed in the charging device 900. The LED 92-1 and the LED 92-2 are examples of the notifier that outputs information to be notified to a user from the aerosol generating system 1. Particularly, the LED 92-1 is an example of a first notifier disposed in association with the button 91-1. Specifically, the LED 92-1 is disposed so as to surround the button 91-1. The LED 92-2 is an example of a second notifier disposed in association with the button 91-2. Specifically, the LED 92-2 is disposed so as to surround the button 91-2.

The features described in relation to the button 11-1 and the button 11-2 may be provided similarly to the button 91-1 and the button 91-2. The features described in relation to the LED 12-1 and may be provided similarly to the LED 92-1 and the LED 92-2. In other words, in the description related to the button 11-1, the button 11-2, the LED 12-1, and the LED 12-2, the button 11-1 may be read as the button 91-1, the button 11-2 may be read as the button 91-2, the LED 12-1 may be read as the LED 92-1, and the LED 12-2 may be read as the LED 92-2.

### (3) Supplement

Each of the first operating portion and the second operating portion just needs to be disposed in any one of the inhaler device 100 and the charging device 900. In the above-described example, an example in which both the first operating portion and the second operating portion are disposed in the inhaler device 100 and an example in which both the first operating portion and the second operating portion are disposed in the charging device 900 have been described; however, the present disclosure is not limited to these examples.

One of the first operating portion and the second operating portion may be disposed in the inhaler device 100, and the charging device 900 may be disposed in the other. For example, the single button 11 may be disposed in the inhaler device 100 as the first operating portion. The single LED 12 may be disposed in the inhaler device 100 as the first notifier. Then, the single button 91 may be disposed in the charging device 900 as the second operating portion. The single LED 92 may be disposed in the charging device 900 as the second notifier. In this case, when the button 11 is depressed, the inhaler device 100 heats the stick substrate 150 in accordance with the first heating profile and notifies information indicating the progress of heating operation with the LED 12. On the other hand, when the button 91 is depressed, the inhaler device 100 heats the stick substrate 150 in accordance with the second heating profile and notifies information indicating the progress of heating operation with the LED 92.

In the above description, an example in which two of each of the button 11 and the LED 12 are disposed in the inhaler device 100 has been described; however, the present disclosure is not limited to this example. Three or more of each of the button 11 and the LED 12 may be disposed. The same applies to the button 91 and the LED 92 disposed in the charging device 900.

### 2.3. Third modification

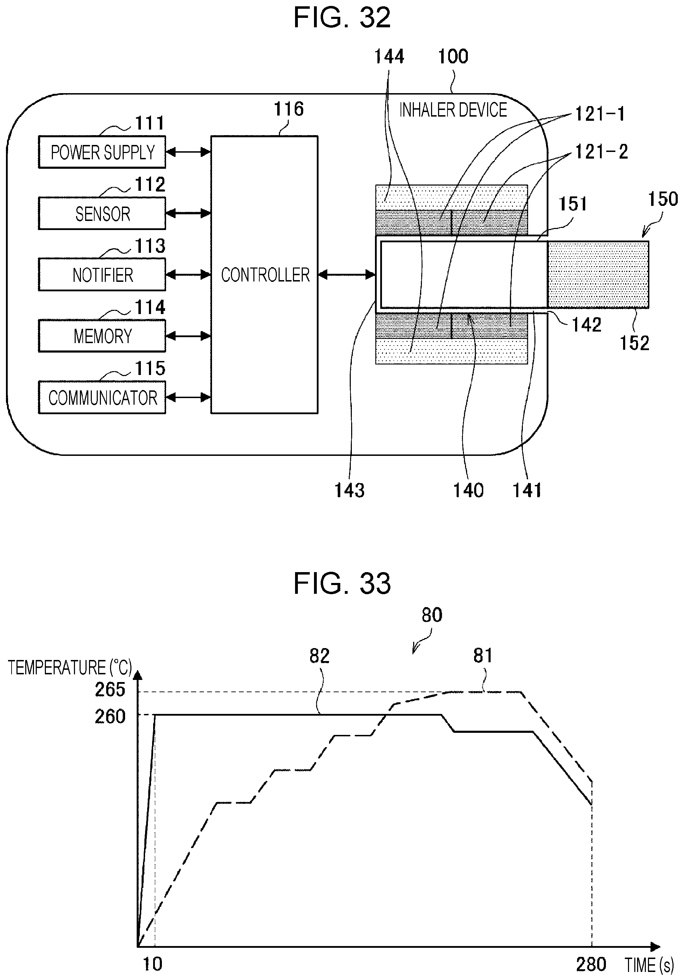

Fig. 32 is a schematic diagram that schematically illustrates an example of the configuration of the inhaler device 100 according to the present modification. As illustrated in Fig. 32, the inhaler device 100 according to the present modification may include two heaters 121, that is, a heater 121-1 and a heater 121-2. In the container 140, a side close to the bottom 143 is also referred to as upstream side, and a side close to the opening 142 is also referred to as downstream side. This is because airflow is generated from the upstream side toward the downstream side at the time when a puff is taken. As illustrated in Fig. 32, the heater 121-1 is disposed on the upstream side. The heater 121-2 is disposed on the downstream side. The controller 116 may control the operation of the heater 121-1 and the operation of the heater 121-2 in accordance with different heating profiles. Examples of heating profiles used to control the operation of the heater 121-1 and the operation of the heater 121-2 will be described with reference to Fig. 33.

Fig. 33 is a graph that schematically illustrates examples of the heating profiles according to the present modification. The abscissa axis of a graph 80 represents time. The ordinate axis of the graph 80 represents temperature. A line 81 represents time-series changes in target temperature, defined in the heating profile used to control the operation of the heater 121-1. The heating profile represented by the line 81 is also referred to as third heating profile. A line 82 represents time-series changes in target temperature, defined in the heating profile used to control the operation of the heater 121-2. The heating profile represented by the line 82 is also referred to as fourth heating profile. The controller 116 may control the operation of the heater 121-1 in accordance with the third heating profile and may control the operation of the heater 121-2 in accordance with the fourth heating profile.

As represented by the line 81 and the line 82, the heater 121-2 disposed on the downstream side initially increases in temperature, and then the heater 121-1 disposed on the upstream side increases in temperature later. With this configuration, an aerosol source is heated in order from a downstream-side part to an upstream-side part of the substrate 151, and an aerosol is generated. If the upstream-side part of the substrate 151 is heated in advance of the downstream-side part, an aerosol generated on the upstream side may be cooled to condense at the time of passing through the downstream-side part. In this case, the downstream-side part of the substrate 151 not yet heated gets wet, with the result that a flavor to be tasted by the user when the downstream-side part of the substrate 151 is heated can degrade. In terms of this point, with the above configuration, a generated aerosol does not pass through an unheated part in the substrate 151. Thus, an unheated part of the substrate 151 is prevented from getting wet, so it is possible to prevent degradation of a flavor to be tasted by the user.

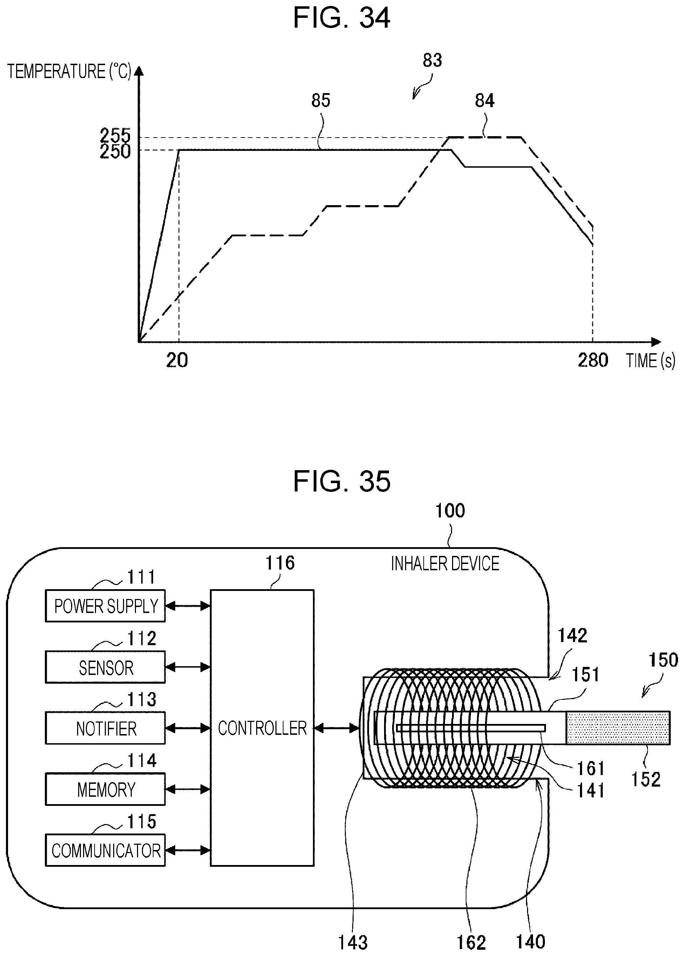

As described in the embodiment, the inhaler device 100 may switch the heating profile to be used at the time when the stick substrate 150 is heated. In an example, the inhaler device 100 can select a heating profile to be used to control the operation of the heater 121-1 from between the third heating profile and a fifth heating profile. The inhaler device 100 can select a heating profile to be used to control the operation of the heater 121-2 from between the fourth heating profile and a sixth profile. Examples of the fifth heating profile and the sixth heating profile will be described with reference to Fig. 34.

Fig. 34 is a graph that schematically illustrates examples of the heating profiles according to the present modification. The abscissa axis of a graph 83 represents time. The ordinate axis of the graph 83 represents temperature. A line 84 represents time-series changes in target temperature, defined in the fifth heating profile used to control the operation of the heater 121-1. A line 85 represents time-series changes in target temperature, defined in the sixth heating profile used to control the operation of the heater 121-2. The controller 116 may control the operation of the heater 121-1 in accordance with the fifth heating profile and may control the operation of the heater 121-2 in accordance with the sixth heating profile.

As illustrated in Figs. 33 and 34, when the fifth and sixth heating profiles are used as well, as in the case where the third and fourth heating profiles are used, the heater 121-2 disposed on the downstream side initially increases in temperature, and then the heater 121-1 disposed on the upstream side increases in temperature later. With the above configuration, it is possible to prevent the degradation of flavor to be tasted by a user.

Furthermore, as illustrated in Figs. 33 and 34, the third heating profile and the fourth heating profile are high-temperature heating profiles, and the fifth and sixth heating profiles are low-temperature heating profiles. For example, the target temperature reaches up to 265°C in the third heating profile, whereas the target temperature reaches up to 255°C in the fifth heating profile. The target temperature reaches 260°C 10 seconds after the start of heating operation in the fourth heating profile, whereas the target temperature reaches 250°C 210 seconds after the start of heating operation in the sixth heating profile. In this way, when the high-temperature heating profile and the low-temperature heating profile are allowed to be switched, a user is able to enjoy smoking feeling according to his or her feeling.

As described in the embodiment, the inhaler device 100 may switch the heating profile to be used during heating operation based on the heating profile. In an example, the inhaler device 100 may switch the heating profiles to be used to the fifth and sixth heating profiles during heating operation based on the third and fourth heating profiles. Of course, it is also possible vice versa. As described in the embodiment, when the controller 116 switches the heating profiles to be used, the controller 116 takes over a heating time before switching and controls the operation of the heater 121-1 and the operation of the heater 121-2 by referencing the switched heating profiles midway.

### 2.4. Fourth modification

As described with reference to Fig. 4, means for atomizing the aerosol source is not limited to heating operation with the heater 121. For example, the means for atomizing the aerosol source may be induction heating. An example of the configuration of the inhaler device 100 that performs induction heating will be described with reference to Fig. 35.

Fig. 35 is a schematic diagram that schematically illustrates an example of the configuration of the inhaler device according to the present modification. As illustrated in Fig. 35, the inhaler device 100 according to the present configuration example includes a power supply 111, a sensor 112, a notifier 113, a memory 114, a communicator 115, a controller 116, a container 140, and an electromagnetic induction source 162.

The configuration of each of the power supply 111, the sensor 112, the notifier 113, the memory 114, the communicator 115, and the controller 116 is substantially the same as the configuration described with reference to Fig. 4. However, the power supply 111 may supply direct current to other structural elements. Other than the above, the power supply 111 may supply alternating current converted by an inverter circuit to other structural elements.

The stick substrate 150 includes the substrate 151 and the inhalation port 152, as described with reference to Fig. 4. The stick substrate 150 further includes the susceptor 161. The susceptor 161 produces heat by electromagnetic induction. The susceptor 161 is made of a conductive material, such as metal. Furthermore, the susceptor 161 is desirably magnetic. In an example, the susceptor 161 can be a metal sheet or a metal rod. The susceptor 161 is disposed in thermal proximity to the aerosol source. In other words, the susceptor 161 is disposed at a position where heat generated at the susceptor 161 is transferred to the aerosol source. In the example illustrated in FIG. 35, the susceptor 161 is included in the substrate 151 of the stick substrate 150. The susceptor 161 may be untouchable from outside of the stick substrate 150. For example, the susceptor 161 may be distributed in a central part of the stick substrate 150 but does not need to be distributed near the outer circumference of the stick substrate 150.

The electromagnetic induction source 162 inductively heats the susceptor 161. When alternating current is applied to the electromagnetic induction source 162, the electromagnetic induction source 162 generates a varying magnetic field (more specifically, an alternating magnetic field). The electromagnetic induction source 162 is disposed at a position where a generated varying magnetic field overlaps the internal space of the container 140, more specifically, a generated varying magnetic field overlaps the susceptor 161 of the stick substrate 150 accommodated in the container 140. The electromagnetic induction source 162 is, for example, a coiled conductive wire wound around the outer circumference of the container 140. Thus, when a varying magnetic field is generated in a state where the stick substrate 150 is accommodated in the container 140, the varying magnetic field generated from the electromagnetic induction source 162 enters the susceptor 161 located in the internal space 141 of the container 140 and inductively heats the susceptor 161. More specifically, an eddy current loss occurs in the susceptor 161, a magnetic hysteresis loss further occurs in the susceptor 161 when the susceptor 161 is magnetic, and the temperature of the susceptor 161 increases. Then, the aerosol source included in the stick substrate 150 is heated and atomized by the inductively heated susceptor 161 to generate an aerosol. In an example, electric power may be supplied to the electromagnetic induction source 162 when the sensor 112 detects a start of user's inhalation and/or an input of predetermined information. Subsequently, the supply of the electric power to the electromagnetic induction source 162 may be stopped when the sensor 112 detects an end of user's inhalation and/or an input of predetermined information.

The configuration example of the inhaler device 100 has been described above. The inhaler device 100 is not limited to the above configuration, and may be configured in various ways as exemplified below.

The susceptor 161 may be provided in the inhaler device 100 instead of being included in the stick substrate 150. In an example, the inhaler device 100 may have the susceptor 161 disposed outside the internal space 141. Specifically, the container 140 may be made of a conductive and magnetic material and function as the susceptor 161. The container 140 serving as the susceptor 161 contacts with the outer circumference of the substrate 151, so the container 140 can be in thermal proximity to an aerosol source included in the substrate 151. In another example, the inhaler device 100 may have the susceptor 161 disposed inside the internal space 141. Specifically, the susceptor 161 formed in a blade shape may be disposed so as to protrude from the bottom 143 of the container 140 into the internal space 141. When the stick substrate 150 is inserted in the internal space 141 of the container 140, the blade-shaped susceptor 161 is inserted into the stick substrate 150 so as to stick into the substrate 151 of the stick substrate 150. Thus, the blade-shaped susceptor 161 can be in thermal proximity to an aerosol source included in the substrate 151.

When the stick substrate 150 has the electromagnetic induction source 162, insertion of the stick substrate 150 may be detected in accordance with a characteristic change of a circuit in the inhaler device 100, which occurs as a result of insertion of the stick substrate 150. An example of the characteristic change of a circuit in the inhaler device 100 is a change in inductance, which occurs in the electromagnetic induction source 162.

In the present modification, the cap 20 is desirably made of a material that is not conductive or magnetic. Examples of such a material include glass, rubber, and plastic. With the above configuration, it is possible to make it difficult for the cap 20 to be inductively heated, so it is possible to ensure the safety of a user.

The electromagnetic induction source 162 according to the present modification corresponds to the heater 121 described in the embodiment. A temperature at which an aerosol source is heated in the present modification corresponds to the temperature of the susceptor 161. The temperature of the susceptor 161 can be estimated in accordance with the electric resistance value of the electromagnetic induction source. In a modification, a parameter related to a temperature at which an aerosol source is heated, defined in a heating profile, is a target value of the temperature (that is, a target temperature) of the susceptor 161. The controller 116 controls the operation of the electromagnetic induction source 162 such that the temperature of the susceptor 161 changes similarly to the target temperature defined in a heating profile.

### 2.5. Fifth modification

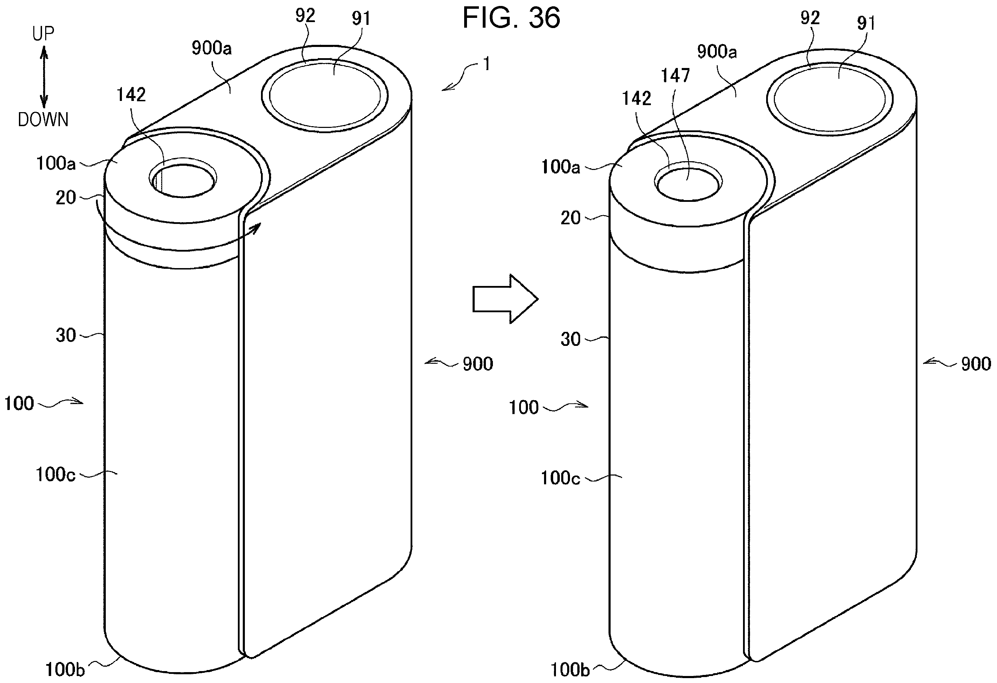

Fig. 36 is a view for illustrating the outline of the aerosol generating system 1 according to the present modification. As illustrated in Fig. 36, the aerosol generating system 1 according to the present modification has a similar external configuration to the embodiment described with reference to Fig. 1. However, in the present modification, the cap 20 can be rotated in a clockwise direction or in a counterclockwise direction, and a shutter 147 operates according to the rotation of the cap 20. As illustrated at the left side of Fig. 36, when the cap 20 is rotated in the counterclockwise direction, the shutter 147 closes the opening 142 as illustrated at the right side of Fig. 36. On the other hand, when the cap 20 is rotated in the clockwise direction, the shutter 147 opens the opening 142. In this way, the shutter 147 may open or close the opening 142 according to the rotation of the cap 20.

The controller 116 may control the operation of the inhaler device 100 according to the status of the shutter 147. In an example, the controller 116 may prohibit heating operation with the heater 121 when the shutter 147 closes the opening 142. On the other hand, the controller 116 may permit heating operation with the heater 121 when the shutter 147 opens the opening 142. Since the stick substrate 150 can be inserted when the shutter 147 opens the opening 142, it is possible to prevent so-called no-substrate heating with the above configuration.

In the present modification, the cap 20 and the body 30 may be detachable or may be formed integrally. The inhaler device 100 and the charging device 900 may be detachable or may be formed integrally.

### 3. Supplement

The preferred embodiment of the present disclosure has been described in detail with reference to the attached drawings, however, the present disclosure is not limited to those examples. It is obvious that persons having ordinary skill in the art in the field of technology to which the present disclosure belongs can conceive of various modifications or alterations within the scope of the technical idea recited in the claims, and these can also be naturally interpreted as belonging to the technical scope of the present disclosure.

In the above description, an example in which a parameter related to a temperature at which an aerosol source is heated, defined in a heating profile, is a target temperature of the heater 121 or the susceptor 161 has been described; however, the present disclosure is not limited to this example. The parameter related to a temperature at which an aerosol source is heated is not only the temperature of the heater 121 itself but also the electric resistance value of the heater 121. The parameter related to a temperature at which an aerosol source is heated is not only the temperature of the susceptor 161 itself but also the electric resistance value of the electromagnetic induction source 162.

In the above description, an example in which connection of the inhaler device 100 with the charging device 900 and release of connection or connection of the cap 20 with the body 30 and release of connection are detected by a magnetic sensor has been described; however, the present disclosure is not limited to the example. Connection of the devices and release of connection may be detected with a selected means, such as whether energization is performed, capacitance, and reading an RF tag of an RFID.

A series of pieces of processing executed by the devices described in the specification may be implemented by any one of software, hardware, and a combination of software and hardware. Programs that are components of software are prestored in, for example, recording media (more specifically, non-transitory computer-readable storage media) provided inside or outside the devices. The programs are, for example, loaded onto a RAM when a computer that controls the devices described in the specification runs the programs and are run on a processing circuit, such as a CPU. Examples of the storage media include a magnetic disk, an optical disk, a magneto-optical disk, and a flash memory. The computer programs may be distributed via, for example, a network, without using storage media. The computer may be an application specific integrated circuit (ASIC), a general-purpose processor that executes a function by loading a software program, a computer on a server used for cloud computing, or the like. A series of pieces of processing executed by the devices described in the specification may be processed by a plurality of computers in a distributed manner.

Pieces of processing described by using the flowchart and the sequence diagram in the specification may be not necessarily executed in order as illustrated. Some processing steps may be executed in parallel. An additional processing step may be adopted, and one or some of the processing steps may be omitted.

The following configurations also belong to the technical scope of the present disclosure.
(1) An aerosol generating system including: a first device, a second device, and a control device, wherein
   the first device includes
      a heater that heats an aerosol source included in a substrate set in the first device, in accordance with a heating profile defining time-series changes in parameter related to a temperature at which the aerosol source is heated, and
      a first power supply that supplies electric power to the heater,
   the second device includes
      a second power supply that supplies electric power to at least any one of the heater and the first power supply in a state where the first device and the second device are connected, and
   when the heater performs heating in a state where the first device and the second device are connected, the control device selects a power supply source to the heater from between the first power supply and the second power supply in accordance with an amount of electric power stored in the first power supply.
(2) In the aerosol generating system according to (1), when the amount of electric power stored in the first power supply is less than a threshold, the control device selects the second power supply as a power supply source to the heater in at least part of a period during which an operation of the heater is controlled in accordance with the heating profile.
(3) In the aerosol generating system according to (2), in a period other than the at least part of the period during which the operation of the heater is controlled in accordance with the heating profile, the control device selects the first power supply as a power supply source to the heater when a predetermined condition is satisfied and selects the second power supply as a power supply source to the heater when the predetermined condition is not satisfied.
(4) In the aerosol generating system according to (3), the predetermined condition includes a condition that connection of the first device with the second device is released.
(5) In the aerosol generating system according to (3) or (4), the predetermined condition is a condition that the amount of electric power stored in the first power supply is greater than or equal to the threshold.
(6) In the aerosol generating system according to any one of (2) to (5),
   the period during which the operation of the heater is controlled in accordance with the heating profile sequentially includes
      an initial temperature rise period during which the temperature at which the aerosol source is heated increases from an initial temperature,
      an intermediate temperature fall period during which the temperature at which the aerosol source is heated decreases, and
      a re-temperature rise period during which the temperature at which the aerosol source is heated increases again, and
   the at least part of the period includes the initial temperature rise period.
(7) In the aerosol generating system according to any one of (2) to (6),
   the control device
   before heating operation based on the heating profile, sets the threshold to a value at which heating is allowed to be continued from a beginning to an end of the period during which the operation of the heater is controlled in accordance with the heating profile, and
   during heating operation based on the heating profile, sets the threshold to a value at which heating is allowed to be continued to an end of a remaining period of the period during which the operation of the heater is controlled in accordance with the heating profile.
(8) In the aerosol generating according to any one of (2) to (7),
   the aerosol generating system further includes a notifier that notifies a user of information, and
   when the amount of electric power stored in the first power supply is greater than or equal to the threshold, the notifier notifies information indicating that connection of the first device with the second device is allowed to be released.
(9) In the aerosol generating system according to (8),
   the aerosol generating system includes the notifier provided in the first device and the notifier provided in the second device,
   when the first device and the second device are connected, the notifier provided in the second device notifies information indicating a status of the first device, and
   when the first device and the second device are not connected, the notifier provided in the first device notifies information indicating a status of the first device.
(10) In the aerosol generating system according to (9), the information indicating the status of the first device relates to at least any one of a progress of heating operation with the heater, a state of charge of the first power supply, and an error having occurred in the first device.
(11) In the aerosol generating system according to any one of (1) to (10), when the second power supply is selected as a power supply source to the heater, the second power supply supplies electric power to both the heater and the first power supply.
(12) In the aerosol generating system according to any one of (1) to (11), in a state where the first device and the second device are connected, the control device sets in accordance with a user operation to the second device whether the second power supply supplies electric power to the first power supply, supplies electric power to the heater, or supplies electric power to both the first power supply and the heater.
(13) In the aerosol generating system according to any one of (1) to (12), in a state where the first device and the second device are connected, when the substrate is set in the first device, the second power supply starts supplying electric power to the heater.
(14) A control method that is executed by a computer that controls at least any one of a first device and a second device, wherein
   the first device includes
      a heater that heats an aerosol source included in a substrate set in the first device, in accordance with a heating profile defining time-series changes in parameter related to a temperature at which the aerosol source is heated, and
      a first power supply that supplies electric power to the heater,
   the second device includes
      a second power supply that supplies electric power to at least any one of the heater and the first power supply in a state where the first device and the second device are connected, and
   the control method comprises, when the heater performs heating in a state where the first device and the second device are connected, selecting a power supply source to the heater from between the first power supply and the second power supply in accordance with an amount of electric power stored in the first power supply.
(15) A program that is executed by a computer that controls at least any one of a first device and a second device, wherein
   the first device includes
      a heater that heats an aerosol source included in a substrate set in the first device, in accordance with a heating profile defining time-series changes in parameter related to a temperature at which the aerosol source is heated, and
      a first power supply that supplies electric power to the heater,
   the second device includes
      a second power supply that supplies electric power to at least any one of the heater and the first power supply in a state where the first device and the second device are connected, and
   the program causes the computer to, when the heater performs heating in a state where the first device and the second device are connected, select a power supply source to the heater from between the first power supply and the second power supply in accordance with an amount of electric power stored in the first power supply.

### Reference Signs List

- 1: aerosol generating system
- 100: inhaler device (100a: top surface, 100b: bottom surface, 100c: side surface)
- 111: power supply
- 112: sensor
- 113: notifier
- 114: memory
- 115: communicator
- 116: controller
- 121: heater
- 140: container
- 141: internal space
- 142: opening
- 143: bottom (143a: projection, 143b: recess)
- 144: heat insulator
- 145: inner wall (145a: projection, 145b: recess, 145c: planar surface, 145d: curved surface)
- 146: airflow path
- 147: shutter
- 150: stick substrate
- 151: substrate
- 152: inhalation port
- 161: susceptor
- 162: electromagnetic induction source
- 190: airflow
- 200: charging device
- 211: power supply
- 212: sensor
- 213: notifier
- 214: memory
- 215: communicator
- 216: controller
- 900: charging device (900a: top surface, 900c: recessed surface)
- 911: power supply
- 912: sensor
- 913: notifier
- 914: memory
- 915: communicator
- 916: controller
- 11: button
- 12: LED
- 13: magnetic portion
- 14: electrical contact
- 20: cap (20a: top surface, 20b: bottom surface, 20c: side surface)
- 21: thread groove
- 22: through-hole (22a: inner wall)
- 23: opening
- 24: head
- 25: protrusion
- 26: through-hole
- 30: body (30a: top surface, 30b: bottom surface, 30c: side surface)
- 31: screw thread
- 32: protrusion
- 33: closed-end hole (33a: inner wall)
- 34: opening
- 35: nonslip pad
- 36: through-hole
- 37: closed-end hole
- 40: air gap
- 50: proximity sensor
- 91: button
- 92: LED
- 921: light emission region
- 93: magnetic portion
- 94: electrical contact

## Claims

1. An aerosol generating system comprising: a first device, a second device, and a control device, wherein
the first device includes
a heater that heats an aerosol source included in a substrate set in the first device, in accordance with a heating profile defining time-series changes in parameter related to a temperature at which the aerosol source is heated, and
a first power supply that supplies electric power to the heater,
the second device includes
a second power supply that supplies electric power to at least any one of the heater and the first power supply in a state where the first device and the second device are connected, and
when the heater performs heating in a state where the first device and the second device are connected, the control device selects a power supply source to the heater from between the first power supply and the second power supply in accordance with an amount of electric power stored in the first power supply.

2. The aerosol generating system according to claim 1, wherein
when the amount of electric power stored in the first power supply is less than a threshold, the control device selects the second power supply as a power supply source to the heater in at least part of a period during which an operation of the heater is controlled in accordance with the heating profile.

3. The aerosol generating system according to claim 2, wherein
in a period other than the at least part of the period during which the operation of the heater is controlled in accordance with the heating profile, the control device selects the first power supply as a power supply source to the heater when a predetermined condition is satisfied and selects the second power supply as a power supply source to the heater when the predetermined condition is not satisfied.

4. The aerosol generating system according to claim 3, wherein
the predetermined condition includes a condition that connection of the first device with the second device is released.

5. The aerosol generating system according to claim 3 or 4, wherein
the predetermined condition is a condition that the amount of electric power stored in the first power supply is greater than or equal to the threshold.

6. The aerosol generating system according to any one of claims 2 to 5, wherein
the period during which the operation of the heater is controlled in accordance with the heating profile sequentially includes
an initial temperature rise period during which the temperature at which the aerosol source is heated increases from an initial temperature,
an intermediate temperature fall period during which the temperature at which the aerosol source is heated decreases, and
a re-temperature rise period during which the temperature at which the aerosol source is heated increases again, and
the at least part of the period includes the initial temperature rise period.

7. The aerosol generating system according to any one of claims 2 to 6, wherein
the control device
before heating operation based on the heating profile, sets the threshold to a value at which heating is allowed to be continued from a beginning to an end of the period during which the operation of the heater is controlled in accordance with the heating profile, and
during heating operation based on the heating profile, sets the threshold to a value at which heating is allowed to be continued to an end of a remaining period of the period during which the operation of the heater is controlled in accordance with the heating profile.

8. The aerosol generating system according to any one of claims 2 to 7, wherein
the aerosol generating system further comprises a notifier that notifies a user of information, and
when the amount of electric power stored in the first power supply is greater than or equal to the threshold, the notifier notifies information indicating that connection of the first device with the second device is allowed to be released.

9. The aerosol generating system according to claim 8, wherein
the aerosol generating system comprises the notifier provided in the first device and the notifier provided in the second device,
when the first device and the second device are connected, the notifier provided in the second device notifies information indicating a status of the first device, and
when the first device and the second device are not connected, the notifier provided in the first device notifies information indicating a status of the first device.

10. The aerosol generating system according to claim 9, wherein
the information indicating the status of the first device relates to at least any one of a progress of heating operation with the heater, a state of charge of the first power supply, and an error having occurred in the first device.

11. The aerosol generating system according to any one of claims 1 to 10, wherein
when the second power supply is selected as a power supply source to the heater, the second power supply supplies electric power to both the heater and the first power supply.

12. The aerosol generating system according to any one of claims 1 to 11, wherein
in a state where the first device and the second device are connected, the control device sets in accordance with a user operation to the second device whether the second power supply supplies electric power to the first power supply, supplies electric power to the heater, or supplies electric power to both the first power supply and the heater.

13. The aerosol generating system according to any one of claims 1 to 12, wherein
in a state where the first device and the second device are connected, when the substrate is set in the first device, the second power supply starts supplying electric power to the heater.

14. A control method that is executed by a computer that controls at least any one of a first device and a second device, wherein
the first device includes
a heater that heats an aerosol source included in a substrate set in the first device, in accordance with a heating profile defining time-series changes in parameter related to a temperature at which the aerosol source is heated, and
a first power supply that supplies electric power to the heater,
the second device includes
a second power supply that supplies electric power to at least any one of the heater and the first power supply in a state where the first device and the second device are connected, and
the control method comprises, when the heater performs heating in a state where the first device and the second device are connected, selecting a power supply source to the heater from between the first power supply and the second power supply in accordance with an amount of electric power stored in the first power supply.

15. A program that is executed by a computer that controls at least any one of a first device and a second device, wherein
the first device includes
a heater that heats an aerosol source included in a substrate set in the first device, in accordance with a heating profile defining time-series changes in parameter related to a temperature at which the aerosol source is heated, and
a first power supply that supplies electric power to the heater,
the second device includes
a second power supply that supplies electric power to at least any one of the heater and the first power supply in a state where the first device and the second device are connected, and
the program causes the computer to, when the heater performs heating in a state where the first device and the second device are connected, select a power supply source to the heater from between the first power supply and the second power supply in accordance with an amount of electric power stored in the first power supply.
